# EUROPÄISCHE PATENTSCHRIFT

(11) **EP 1 866 280 B1**
(45) Veröffentlichungstag und Bekanntmachung des Hinweises auf die Patenterteilung: **13.08.2008**
(21) Anmeldenummer: 06723338.7
(22) Anmeldetag: 10.03.2006
(51) Int. Cl.: C07C 243/40, C07C 255/57, C07C 243/38, C07C 323/62, A61P 3/00, A61K 31/166

(54) **ACYLHYCLRAZIDE ALS KINASE INHIBITOREN INSBESONDERE FÜR SGK**
ACYL HYDRAZIDES AS KINASE INHIBITORS, IN PARTICULAR FOR SGK
ACYLHYDRAZIDES UTILISES COMME INHIBITEUR DE KINASE, NOTAMMENT DE LA SGK

(30) Priorität: 04.04.2005 DE 102005015255
(43) Veröffentlichungstag der Anmeldung: 19.12.2007
(73) Patentinhaber: Merck Patent GmbH, 64293 Darmstadt (DE)
(72) Erfinder: GERICKE, Rolf, 64342 Seeheim-Jugenheim (DE); DORSCH, Dieter, 64372 Ober-Ramstadt (DE); MEDERSKI, Werner, 64673 Zwingenberg (DE); KLEIN, Markus, 64331 Weiterstadt (DE); BEIER, Norbert, 64354 Reinheim (DE); LANG, Florian, 72076 Tuebingen (DE)
(86) Internationale Anmeldenummer: PCT/EP2006/002220
(87) Internationale Veröffentlichungsnummer: WO 2006/105850

(56) Entgegenhaltungen:
- WO-A-00/62781
- WO-A-01/14412

## Beschreibung

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der zellvolumenregulierten humanen Kinase h-sgk (human serum and glucocorticoid dependent kinase oder SGK) eine Rolle spielt, ferner pharmazeutische Zusammensetzungen, die diese Verbindungen enthalten, sowie die Verwendung der Verbindungen zur Behandlung SGKbedingter Krankheiten.

Die SGK mit den lsoformen SGK-1, SGK-2 und SGK-3 sind eine Serin/Threonin-Proteinkinase Familie (WO 02/17893). Die erfindungsgemäßen Verbindungen sind vorzugsweise selektive Inhibitoren der SGK-1. Ferner können sie Inhibitoren der SGK-2 und/oder SGK-3 sein.

Im einzelnen betrifft die vorliegende Erfindung Verbindungen, die die Signaltransduktion der SGK hemmen, regulieren und/oder modulieren, Zusammensetzungen, die diese Verbindungen enthalten, sowie Verfahren zu ihrer Verwendung zur Behandlung von SGK-bedingten Krankheiten und Leiden wie Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).
Die erfindungsgemäßen Verbindungen können auch das Wachstum von Tumorzellen und Tumormetastasen hemmen und sind deshalb für die Tumortherapie geeignet.
Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.
Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden. Darüberhinaus wirken die erfindungsgemäßen Verbindungen der Zellalterung und Stress entgegen und steigern somit die Lebenserwartung und die Fitness im Alter.
Die erfindungsgemäßen Verbindungen finden ferner Verwendung bei der Behandlung von Tinitus.

Die Identifikation von kleinen Verbindungen, die die Signaltransduktion der SGK spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.
insbesondere zeigen sie inhibierende Eigenschaften der SGK.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit yATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemolumineszenz nachweisbar (Ross et al., Biochem. J., 2002, 366, 977-981).

### STAND DER TECHNIK

In der WO 00/62781 ist die Verwendung von Arzneimitteln enthaltend Hemmstoffe der zellvolumenregulierten humanen Kinase H-SGK beschrieben.
Benzyliden-benzohydrazide mit antibakterieller Wirkung sind in der WO 02/070464 A2 beschrieben. Die Verwendung von Acylhydraziden zur Behandlung bakterieller Infektionen ist in WO 01/70213 offenbart.
Andere Acylhydrazonderivate, u.a. zur Behandlung von Diabeteskomplikationen, sind in JP 11-106371 offenbart.
Methoxysubstituierte aromatische Acylhydrazonderivate zur Behandlung von Krebs sind von T.Kametani et al. in Yakugaku Zasshi (1963), 83, 851-855 und in Yakugaku Zasshi (1963), 83, 844-847 beschrieben. Andere aromatische Acylhydrazonderivate als Verstärker von Sedativa und zur Blutdrucksenkung sind in JP 41-20699 offenbart.

Die Verwendung von Kinase-Inhibitoren in der antiinfektiösen Therapie ist von C.Doerig in Cell. Mol. Biol. Lett. Vol.8, No. 2A, 2003, 524-525 beschrieben.
Die Verwendung von Kinase-Inhibitoren bei Fettsucht ist von N.Perrotti in J. Biol. Chem. 2001, März 23; 276(12):9406-9412 beschrieben.

In nachstehenden Literaturstellen wird die Verwendung von SGK-Hemmern bei der Behandlung von Krankheiten nahegelegt und/oder beschrieben:

1: Chung EJ, Sung YK, Farooq M, Kim Y, Im S, Tak WY, Hwang YJ, Kim YI, Han HS, Kim JC, Kim MK. Gene expression profile analysis in human hepatocellular carcinoma by cDNA microarray. Mol Cells. 2002;14:382-7.

2: Brickley DR, Mikosz CA, Hagan CR, Conzen SD. Ubiquitin modification of serum and glucocorticoid-induced protein kinase-1 (SGK-1). J Biol Chem. 2002;277:43064-70.

3: Fillon S, Klingel K, Warntges S, Sauter M, Gabrysch S, Pestel S, Tanneur V, Waldegger S, Zipfel A, Viebahn R, Haussinger D, Broer S, Kandolf R, Lang F. Expression of the serine/threonine kinase hSGK1 in chronic viral hepatitis. Cell Physiol Biochem. 2002;12:47-54.

4: Brunet A, Park J, Tran H, Hu LS, Hemmings BA, Greenberg ME. Protein kinase SGK mediates survival signals by phosphorylating the forkhead transcription factor FKHRL1 (FOX03a). Mol Cell Biol 2001;21:952-65

5: Mikosz CA, Brickley DR, Sharkey MS, Moran TW, Conzen SD. Glucocorticoid receptor-mediated protection from apoptosis is associated with induction of the serine/threonine survival kinase gene, sgk-1. J Biol Chem. 2001;276:16649-54.

6: Zuo Z, Urban G, Scammell JG, Dean NM, McLean TK, Aragon I, Honkanen RE. Ser/Thr protein phosphatase type 5 (PP5) is a negative regulator of glucocorticoid receptor-mediated growth arrest. Biochemistry. 1999;38:8849-57.

7: Buse P, Tran SH, Luther E, Phu PT, Aponte GW, Firestone GL. Cell cycle and hormonal control of nuclear-cytoplasmic localization of the serum- and glucocorticoid-inducible protein kinase, Sgk, in mammary tumor cells. A novel convergence point of anti-proliferative and proliferative cell signalling pathways. J Biol Chem. 1999;274:7253-63.

8: M. Hertweck, C. Göbel, R. Baumeister: C.elegans SGK-1 is the critical component in the Akt/PKB Kinase complex to control stress response and life span. Developmental Cell, Vol. 6, 577-588, April, 2004.

### ZUSAMMENFASSUNG DER ERFINDUNG

Die Erfindung betrifft Verbindungen der Formel I 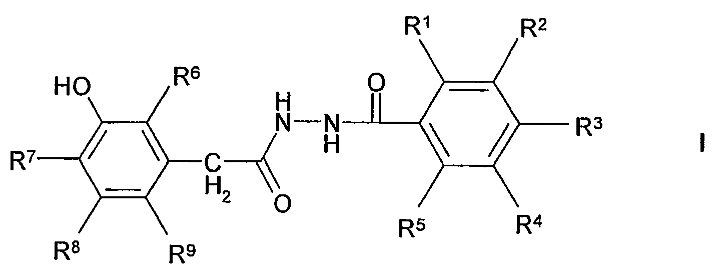 worin
- R¹, R², R³, R⁴, R⁵, R⁶, R⁷, R⁸, R⁹: jeweils unabhängig voneinander H, A, OSO₂A, Hal, NO₂, OR¹⁰, N(R¹⁰)₂, CN, -[C(R¹⁰)₂]ₙCOOR¹⁰, O-[C(R¹⁰)₂]ₒCOOR¹⁰, SO₃H, -[C(R¹⁰)₂]ₙAr, -CO-Ar, O-[C(R¹⁰)₂]ₙAr, -[C(R¹⁰)₂]ₙHet, -[C(R¹⁰)₂]ₙC≡CH, O-[C(R¹⁰)₂]ₙC≡CH, -[C(R¹⁰)₂]ₙCON(R¹⁰)₂, -[C(R¹⁰)₂]ₙCONR¹⁰N(R¹⁰)₂, O-[C(R¹⁰)₂]ₙCON(R¹⁰)₂, O-[C(R¹⁰)₂]ₒCONR¹⁰N(R¹⁰)₂, NR¹⁰COA, NR¹⁰CON(R¹⁰)₂, NR¹⁰SO₂A, N(SO₂A)₂, COR¹⁰, S(O)ₘAr, SO₂NR¹⁰ oder S(O)ₘA,
- R¹ und R², R² und R³, R³ und R⁴ oder R⁴ und R⁵: zusammen auch CH=CH-CH=CH,
- A: unverzweigtes oder verzweigtes Alkyl mit 1-6 C-Atomen, worin 1-7 H-Atome durch F ersetzt sein können, oder cylisches Alkyl mit 3-7 C-Atomen,
- Ar: unsubstituiertes oder ein-, zwei- oder dreifach durch Hal, A, OR¹⁰, N(R¹⁰)₂, NO₂, CN, Phenyl, CON(R¹⁰)₂, NR¹⁰COA, NR¹⁰CON(R¹⁰)₂, NR¹⁰SO₂A, COR¹⁰, SO₂N(R¹⁰)₂, S(O)ₘA, -[C(R¹⁰)₂]ₙ-COOR¹⁰ und/oder -O[C(R¹⁰)₂]ₒ-COOR¹⁰ substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der ein-, zwei- oder dreifach durch Hal, A, OR¹⁰, N(R¹⁰)₂, NO₂, CN, COOR¹⁰, CON(R¹⁰)₂, NR¹⁰COA, NR¹⁰SO₂A, COR¹⁰, SO₂NR¹⁰, S(O)ₘA, =S, =NR¹⁰ und/oder =O (Carbonylsauerstoff) substituiert sein kann,
- R¹⁰: H oder A,
- Hal: F, Cl, Br oder I,
- m: 0, 1 oder 2,
- n: 0, 1, 2 oder 3,
- o: 1, 2 oder 3
bedeuten,
sowie ihre pharmazeutisch verwendbaren Derivate, Salze, Solvate und Stereoisomere, einschließlich deren Mischungen in allen Verhältnissen.

Gegenstand der Erfindung sind die Verbindungen der Formel I und ihre Salze sowie ein Verfahren zur Herstellung von Verbindungen der Formel I nach den Ansprüchen 1-16 sowie ihrer pharmazeutisch verwendbaren Derivate, Solvate, Salze und Stereoisomere, dadurch gekennzeichnet, daß man
a) eine Verbindung der Formel II 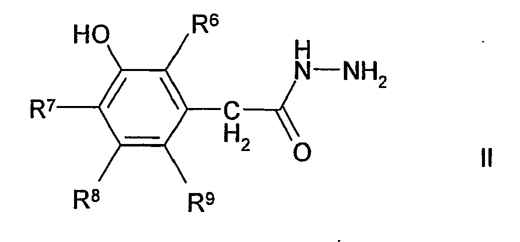 worin
   R⁶, R⁷, R⁸ und R⁹ die in Anspruch 1 angegebenen Bedeutungen haben,
   mit einer Verbindung der Formel III 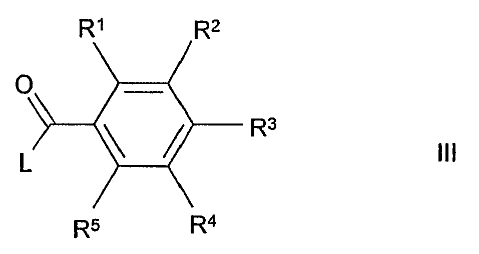 worin
   L Cl, Br, I oder eine freie oder reaktionsfähig funktionell abgewandelte OH-Gruppe bedeutet und
   R¹, R², R³, R⁴ und R⁵ die in Anspruch 1 angegebenen Bedeutungen haben,
   umsetzt,
   oder
b) eine Verbindung der Formel IV 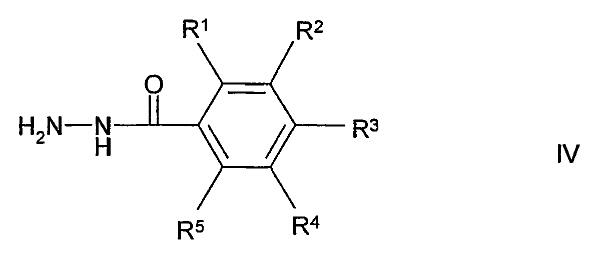 worin
   R¹, R², R³, R⁴ und R⁵ die in Anspruch 1 angegebenen Bedeutungen haben,
   mit einer Verbindung der Formel V 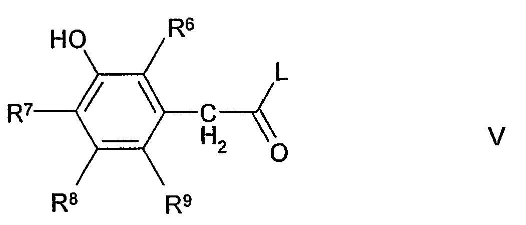 worin
   L Cl, Br, I oder eine freie oder reaktionsfähig funktionell abgewandelte OH-Gruppe bedeutet und
   R⁶, R⁷, R⁸ und R⁹ die in Anspruch 1 angegebenen Bedeutungen haben,
   umsetzt,
   oder
c) in einer Verbindung der Formel I einen Rest R¹, R², R³, R⁴, R⁵, R⁶, R⁷, R⁸ und/oder R⁹ in einen anderen Rest R¹, R², R³, R⁴, R⁵, R⁶, R⁷, R⁸ und/oder R⁹ umwandelt,
   indem man einen Ether durch Hydrolyse oder Hydrogenolyse spaltet,
   und/oder eine Base oder Säure der Formel I in eines ihrer Salze umwandelt.

Gegenstand der Erfindung sind auch die Stereoisomeren (E, Z-lsomeren) sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.
Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, gilt, daß deren Bedeutungen unabhängig voneinander sind.
Vor- und nachstehend haben die Reste bzw. Parameter R¹, R², R³, R⁴, R⁵, R⁶, R⁷, R⁸ und R⁹ die bei der Formel I angegebenen Bedeutungen, falls nicht ausdrücklich etwas anderes angegeben ist.

A bedeutet Alkyl, ist unverzweigt (linear) oder verzweigt, und hat 1, 2, 3, 4, 5 oder 6 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, lsobutyl, sek.-Butyl, tert.-Butyl, Pentyl, Hexyl, Trifluormethyl, Pentafluorethyl oder 1,1,1-Trifluorethyl.

Ac bedeutet Acetyl, Bn bedeutet Benzyl, Ms bedeutet -SO₂CH₃.

R¹ bedeutet vorzugsweise H, A, Hal, NO₂, OR¹⁰, -[C(R¹⁰)₂]ₙAr oder O-[C(R¹⁰)₂]ₙAr, besonders bevorzugt H, A, Hal, NO₂, OH, OCH₃, Phenyl, Benzyl, Phenoxy oder Benzyloxy, ganz besonders bevorzugt OH, Hal oder A.

R² bedeutet vorzugsweise H, A, Hal, CN, NO₂, OR¹⁰, -[C(R¹⁰)₂]ₙAr oder O-[C(R¹⁰)₂]ₙAr, besonders bevorzugt H, A, Hal, CN, NH₂, NO₂, OH, OCH₃, Benzyl, Phenyl, Phenoxy oder Benzyloxy, ganz besonders bevorzugt H, A oder Hai.

R³ bedeutet vorzugsweise H, A, Hal, NO₂, OR¹⁰, -[C(R¹⁰)₂]ₙAr, O-[C(R¹⁰)₂]ₙAr, -[C(R¹⁰)₂]ₙCOOR¹⁰ oder S(O)ₘA, besonders bevorzugt H, A, Hal, NO₂, OH, OCH₃, Phenyl, Benzyl, Phenoxy, Benzyloxy, Methoxycarbonyl, Carboxy oder SA, ganz besonders bevorzugt OH.

R⁴ bedeutet vorzugsweise H, A, Hal, CONH₂, CN, NO₂ oder OR¹⁰, besonders bevorzugt H, A, Hal, CN, CONH₂, NO₂, OH oder OCH₃, ganz besonders bevorzugt H.

R⁵ bedeutet vorzugsweise H, A, Hal, OR¹⁰, -[C(R¹⁰)₂]ₙAr oder O-[C(R¹⁰)₂]ₙAr, besonders bevorzugt H, A, Hal, OH, OCH₃, Phenyl, Benzyl, Phenoxy oder Benzyloxy, besonders bevorzugt H oder OH.

R⁶ bedeutet vorzugsweise H.

R⁷ bedeutet vorzugsweise H oder OR¹⁰, besonders bevorzugt H, OH oder OCH₃, besonders bevorzugt H.

R⁸ bedeutet vorzugsweise H oder OR¹⁰, besonders bevorzugt H, OH oder OCH₃, ganz besonders bevorzugt H.

R⁹ bedeutet vorzugsweise H.

R¹⁰ bedeutet H oder A, vorzugsweise H oder Methyl. R¹⁰ bedeutet ganz besonders bevorzugt H.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methyl-sulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylaminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise z.B. unsubstituiertes oder ein-, zwei- oder dreifach durch Hal, A, OR¹⁰, SO₂A, COOR¹⁰ oder CN substituiertes Phenyl, ganz besonders bevorzugt unsubstituiertes oder ein-, zwei- oder dreifach durch Hal und/oder A substituiertes Phenyl.
Het bedeutet, ungeachtetet weiterer Substitutionen, z.B. 2- oder 3-Furyl, 2- oder 3-Thienyl, 1-, 2- oder 3-Pyrrolyl, 1-, 2, 4- oder 5-lmidazolyl, 1-, 3-, 4- oder 5-Pyrazoiyl, 2-, 4- oder 5-Oxazolyl, 3-, 4- oder 5-isoxazoiyi, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder-5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder - 4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethyiendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet vorzugsweise einen einkernigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann.
Het bedeutet besonders bevorzugt einen einkernigen gesättigten Heterocyclus mit 1 bis 2 N- und/oder O-Atomen, der unsubstituiert oder ein- oder zweifach durch A substituiert sein kann.
In einer weiteren Ausführungsform bedeutet Het ganz besonders bevorzugt Pyrrolidinyl, Piperidinyl, Morpholinyl oder Piperazinyl.
In einer weiteren Ausführungsform bedeutet Het besonders bevorzugt unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis lo ausgedrückt werden, die der Formel I entsprechen und worin die nicht näher bezeichneten Reste die bei der Formel I angegebene Bedeutung haben, worin jedoch

| | | |
|---|---|---|
| in la | R¹ | H, A, Hal, NO₂, OR¹⁰, -[C(R¹⁰)₂]ₙAr oder O-[C(R¹⁰)₂]ₙAr, |
| | bedeutet; | |
| in lb | R² | H, A, Hal, CN, NO₂, OR¹⁰, -[C(R¹⁰)₂]ₙAr oder O-[C(R¹⁰)₂]ₙAr |
| | bedeutet; | |
| in lc | R³ | H, A, Hal, NO₂, OR¹⁰, -[C(R¹⁰)₂]ₙAr, O-[C(R¹⁰)₂]ₙAr, -[C(R¹⁰)₂]ₙCOOR¹⁰ oder S(O)ₘA |
| | bedeutet; | |
| in ld | R⁴ | H, A, Hal, CONH₂, CN, NO₂ oder OR¹⁰ |
| | bedeutet; | |
| in le | R⁵ | H, A, Hal, OR¹⁰, -[C(R¹⁰)₂]ₙAr oder O-[C(R¹⁰)₂]ₙAr |
| | bedeutet; | |
| in lf | R⁶ | H oder A bedeutet; |
| in Ig | R⁷ | H, A oder OR¹⁰ bedeutet; |
| in lh | R⁸ | H, A oder OR¹⁰ bedeutet; |
| in li | R⁹ | H oder A bedeutet; |
| in lj | Ar | unsubstituiertes oder ein-, zwei- oder dreifach durch Hal und/oder A substituiertes Phenyl |
| | bedeutet; | |
| in lk | Het | einen einkernigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann, |
| | bedeutet; | |
| in ll | Het | einen einkernigen gesättigten Heterocyclus mit 1 bis 2 N- und/oder O-Atomen, der unsubstituiert oder ein- oder zwei- fach durch A substituiert sein kann, |
| | bedeutet; | |
| in lm | Het | unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl, |
| | bedeutet; | |
| in ln | R¹ | H, A, Hal, NO₂, OR¹⁰, -[C(R¹⁰)₂]ₙAr oder O-[C(R¹⁰)₂]ₙAr, |
| | R² | H, A, Hal, CN, N(R¹⁰)₂, NO₂, OR¹⁰, -[C(R¹⁰)₂]ₙAr oder O-[C(R¹⁰)₂]ₙAr, |
| | R³ | H, A, Hal, NO₂, OR¹⁰, -[C(R¹⁰)₂]ₙAr, O-[C(R¹⁰)₂]ₙAr, -[C(R¹⁰)₂]ₙCOOR¹⁰ oder S(O)ₘA, |
| | R⁴ | H, A, Hal, CONH₂, CN, NO₂ oder OR¹⁰, |
| | R⁵ | H, A, Hal, OR¹⁰, -[C(R¹⁰)₂]ₙAr oder O-[C(R¹⁰)₂]ₙAr, |
| | R⁶ | H, |
| | R⁷ | H oder OR¹⁰, |
| | R⁸ | H oder OR¹⁰, |
| | R⁹ | H, |
| | R¹ und R², R² und R³, R³ und R⁴ oder R⁴ und R⁵ | zusammen auch CH=CH-CH=CH, |
| | A | unverzweigtes oder verzweigtes Alkyl mit 1-6 C-Atomen, worin 1-7 H-Atome durch F ersetzt sein können, |
| | Ar | unsubstituiertes oder ein-, zwei- oder dreifach durch Hal und/oder A substituiertes Phenyl, |
| | R¹⁰ | H oder A, |
| | Hal | F, Cl, Br oder I, |
| | m | 0, 1 oder 2, |
| | n | 0, 1, 2 oder 3 |
| | bedeuten; | |
| in lo | R¹ | OH, A oder Hal, |
| | R² | H, A oder Hal, |
| | R³ | OH, |
| | R⁴ | H, A oder Hal, |
| | R⁵ | H oder OH, |
| | R⁶ | H, |
| | R⁷ | H, |
| | R⁸ | H, |
| | R⁹ | H, |
| | R¹ und R², R² und R³, R³ und R⁴ oder R⁴ und R⁵ | zusammen auch CH=CH-CH=CH, |
| | A | unverzweigtes oder verzweigtes Alkyl mit 1-6 C-Atomen, worin 1-7 H-Atome durch F ersetzt sein können, |
| | Hal | F, Cl, Br oder I |
| | bedeuten; | |

sowie ihre pharmazeutisch verwendbaren Derivate, Solvate, Salze und Stereoisomere, einschließlich deren Mischungen in allen Verhältnissen.

Besonders bevorzugt sind die Verbindungen der Formel I ausgewählt aus der Gruppe

| Nr. | Strukturformel | F. [°C] |
|---|---|---|
| 1 | 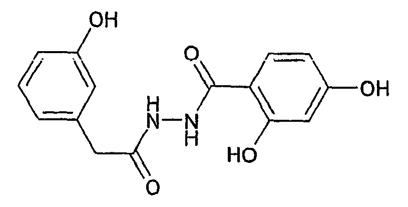 | 233-235 |
| 57 | 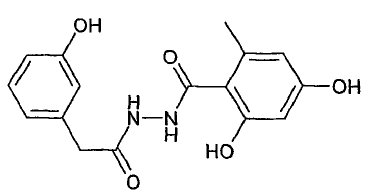 | 226-227 |
| | | |
| 60 | 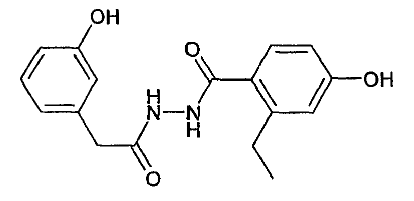 | 199-200 |
| 61 | 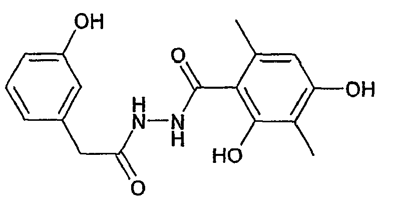 | 193-194 |
| 62 | 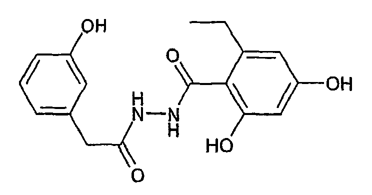 | 251 |
| 63 | 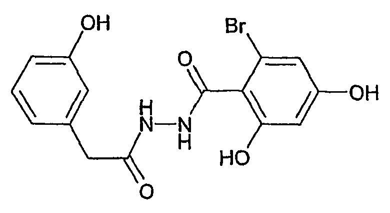 | 226-227 |
| 68 | 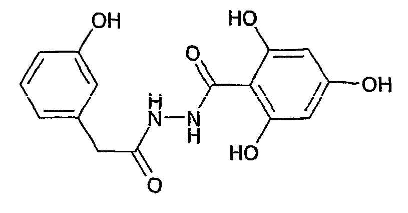 | 237-238 |
| 69 | 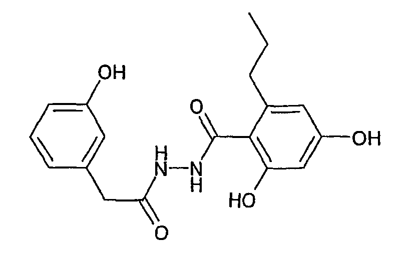 | 198-200 |
| 70 | 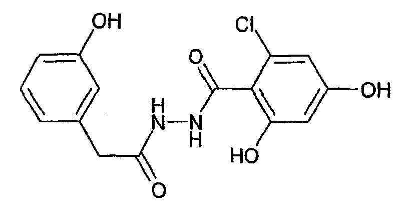 | 213-215 |
| 72 | 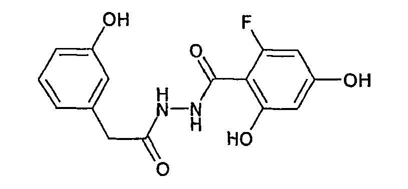 | 230-232 |
| 73 | 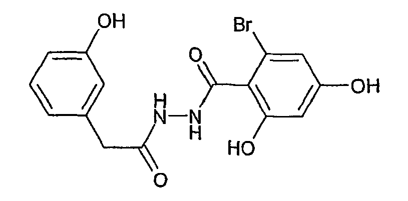 | 259 |
| 84 | 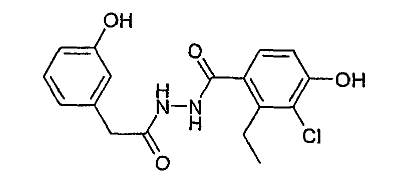 | 259 |
| 87 | 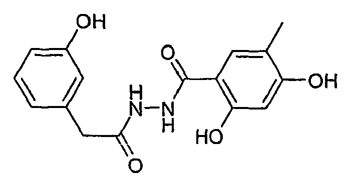 | 240 |

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I können vorzugsweise erhalten werden, indem man ein Hydrazid der Formel II mit einer Verbindung der Formel III umsetzt.
Die Umsetzung erfolgt nach Methoden, die dem Fachmann bekannt sind. Die Umsetzung erfolgt in der Regel in einem inerten Lösungsmittel, gegebenenfalls in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel III.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichiorethan,Tetrachlorkohlenstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Als Lösungsmittel besonders bevorzugt sind Wasser oder DMF.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Verbindungen der Formel I können weiterhin vorzugsweise erhalten werden, indem man ein Hydrazid der Formel IV mit einer Verbindung der Formel V umsetzt.

Die Umsetzung erfolgt in der Regel in einem inerten Lösungsmittel, in Gegenwart eines säurebindenden Mittels vorzugsweise einer organischen Base wie DIPEA, Triethylamin, Dimethylanilin, Pyridin oder Chinolin oder eines Überschusses der Carboxykomponente der Formel V.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

In den Verbindungen der Formel V bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).
Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.
Aktivierte Ester werden zweckmäßig in situ gebildet, z. B. durch Zusatz von HOBt oder N-Hydroxysuccinimid.

Verbindungen der Formel I können ferner erhalten werden, indem man einen Rest R¹, R², R³, R⁴, R⁵, R⁶, R⁷, R⁸ und/oder R⁹ in einen anderen Rest R¹, R², R³, R⁴, R⁵, R⁶, R⁷, R⁸ und/oder R⁹ umwandelt, indem man z.B. einen Ether durch Hydrolyse oder Hydrogenolyse spaltet.

Die Spaltung eines Ethers erfolgt unter Methoden, wie sie dem Fachmann bekannt sind.
Eine Standardmethode zur Etherspaltung, z.B. eines Methylethers, ist die Verwendung von Bortribromid.
Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, lsobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B.

Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieseigei fixierte chiral derivatisierte Methacrylatpolymere). Als Laufmittel eignen sich hierfür wäßrige oder alkoholische Lösungsmittelgemische wie z.B. Hexan/lsopropanol/Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Ais Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nicht-toxischen Vehikel formuliert werden. Lösungsvermittler und Emulgiermittel, wie z.B. ethoxylierte lsostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist.
Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Derivate, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von SGK-bedingten Krankheiten.

Gegenstand der Erfindung ist somit die Verwendung von Verbindungen nach Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.. Bevorzugt ist hierbei SGK.

Bevorzugt ist die Verwendung von Verbindungen gemäß Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, die durch Inhibierung der SGK durch die Verbindungen nach Anspruch 1 beeinflußt werden.

Die vorliegende Erfindung umfasst die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung von Diabetes (z.B. Diabetes mellitus, diabetische Nephropathie, diabetische Neuropathie, diabetische Angiopathie und Mikroangiopathie), Fettsucht, metabolisches Syndrom (Dyslipidämie), systemische und pulmonale Hypertonie, Herzkreislauferkrankungen (z.B. kardiale Fibrosen nach Myokardinfarkt, Herzhypertrophie und Herzinsuffizienz, Arteriosklerose) und Nierenerkrankungen (z.B. Glomerulosklerose, Nephrosklerose, Nephritis, Nephropathie, Störung der Elektrolytausscheidung), allgemein bei jeglicher Art von Fibrosen und entzündlichen Prozessen (z.B. Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer).
Die erfindungsgemäßen Verbindungen können auch das Wachstum von Krebs, Tumorzellen und Tumormetastasen hemmen und sind deshalb für die Tumortherapie geeignet.
Die erfindungsgemäßen Verbindungen finden weiterhin Verwendung zur Behandlung von Koagulopathien, wie z.B. Dysfibrinogenämie, Hypoprokonvertinämie, Hämophilie B, Stuart-Prower-Defekt, ProthrombinKomplex-Mangel, Verbrauchskoagulopathie, Hyperfibrinolyse, Immunokoagulopathie oder komplexer Koagulopathien, wie auch bei neuronaler Erregbarkeit, z.B. Epilepsie. Die erfindungsgemäßen Verbindungen können auch bei der Behandlung eines Glaukoms oder Katarakt therapeutisch eingesetzt werden.
Die erfindungsgemäßen Verbindungen finden ferner Verwendung bei der Behandlung bakterieller Infektionen sowie in einer antiinfektiösen Therapie. Die erfindungsgemäßen Verbindungen können auch zur Steigerung der Lernfähigkeit und Aufmerksamkeit therapeutisch eingesetzt werden.

Bevorzugt ist die Verwendung von Verbindungen gemäß Anspruch 1, sowie ihrer pharmazeutisch verwendbaren Derivate, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress.

Bei Diabetes handelt es sich vorzugsweise um Diabetes mellitus, diabetische Nephropathie, diabetische Neuropathie, diabetische Angiopathie und Mikroangiopathie.

Bei Herzkreislauferkrankungen handelt es sich vorzugsweise um kardiale Fibrosen nach Myokardinfarkt, Herzhypertrophie, Herzinsuffizienz und Arteriosklerose.

Bei Nierenerkrankungen handelt es sich vorzugsweise um Glomerulosklerose, Nephrosklerose, Nephritis, Nephropathie und Störung der Elektrolytausscheidung.

Bei Fibrosen und entzündlichen Prozessen handelt es sich vorzugsweise um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung, Morbus Alzheimer.

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.
- Massenspektrometrie (MS):: El (Elektronenstoß-lonisation) M⁺
FAB (Fast Atom Bombardment) (M+H)⁺
ESI (Electrospray lonization) (M+H)⁺ (wenn nichts anderes angegeben)

### Beispiel 1

Herstellung von 2-Ethyl-4-hydroxy-benzoesäure-*N*'-[2-(3-hydroxy-phenyl)-acetyl]-hydrazid ("60")
1.1

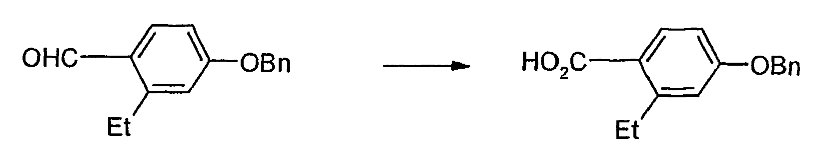

Zu 120 g 4-Benzyloxy-2-ethyl-benzaldehyde, gelöst in 1 I DMSO, wird unter Kühlen und Rühren eine wässrige Lösung (750 ml) von 85 g NaClO₂ und 90 g NaH₂PO₄ getropft, wobei die Temperatur nicht über 35 °C steigen sollte. Es wird noch 3 h bei RT nachgerührt und der gebildete Niederschlag abgesaugt. Der Feststoff wird sodann in 600 ml EtOAc gelöst, getrocknet und wieder eingeengt. Der Rückstand wird aus (Me₂CH)₂O kristallisiert. Man erhält 4-Benzyloxy-2-ethyl-benzoesäure; Ausbeute 96,2 g (75 %); F. 132-133°.
1.2 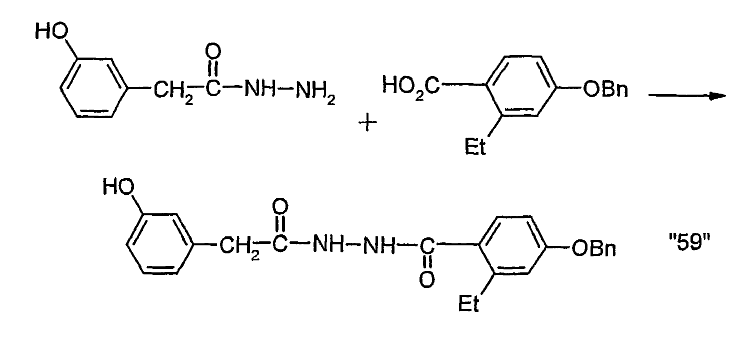 1,6 g 4-Benzyloxy-2-ethyl-benzoesäure wird mit 4 ml SOCl₂ refluxiert bis eine klare Lösung entsteht. Das SOCl₂ wird abgezogen, anschließend wird noch 2 × mit CH₂Cl₂ zur Trockne eingedampft. Das Säurechlorid wird nun in 3 mi DMF gelöst und 1,14 g (3-Hydroxy-phenyl)-essigsäurehydrazid zugefügt. Nach 2-stündigem Rühren bei 40°C wird auf H₂O gegeben und weiter gerührt. Die ausgefallene Substanz wird abgesaugt und getrocknet. Man erhält 4-Benzyloxy-2-ethyl-benzoesäure-N'-[2-(3-hydroxy-phenyl)-acetyl]-hydrazid ("59"); Ausbeute 1,49 g (59 %); F. 190-191°.
1.3 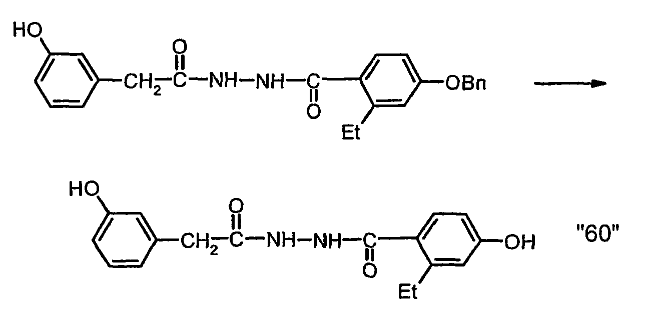 57,4 g 4-Benzyloxy-2-ethyl-benzoesäure-*N*'-[2-(3-hydroxy-phenyl)-acetyl]-hydrazid werden in 1,5 I THF gelöst und 24 h hydriert. Der Katalysator (Pd/C-5%, 35 g) wird in 3 Portionen zugefügt. Der Katalysator wird abgesaugt, die Lösung eingeengt und der Rückstand aus MeCN kristallisiert. Man erhält 41,1 g "60" (92 %); F. 199-200°.

### Beispiel 2

Herstellung von 3,5-Dihydroxy-4'-methyl-biphenyl-2-carbonsäure-*N*'-[2-(3-hydroxy-phenyl)-acetyl]-hydrazid ("65")
2.1

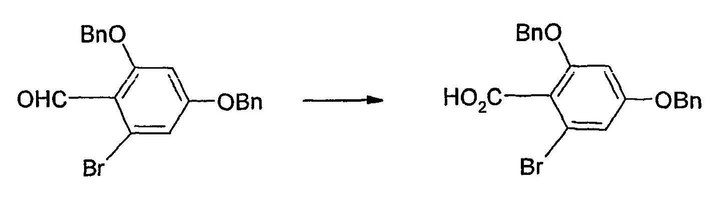

Die Umsetzung erfolgt analog Beispiel 1.1.

Man erhält 2,4-Bis-benzyloxy-6-brom-benzoesäure, F. 152-154°.
2.2 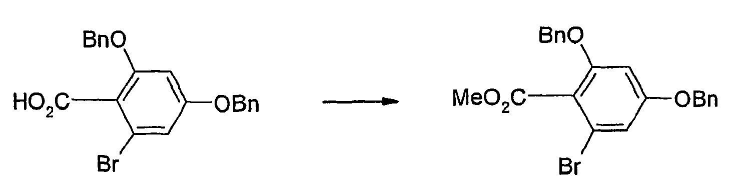 47 g 2,4-Bis-benzyloxy-6-brom-benzoesäure, 9 ml Mel und 40 g K₂CO₃ werden in 150 ml DMF für 2 h bei 50 °C gerührt. Dann wird mit H₂O verdünnt, 3x mit EtOAc extrahiert, getrocknet, eingeengt und der Rückstand mit (Me₂CH)₂O kristallisiert. Ausbeute: 37 g 2,4-Bis-benzyloxy-6-brom-benzoesäure-methylester (76 %); F. 90-91 °.
2.3 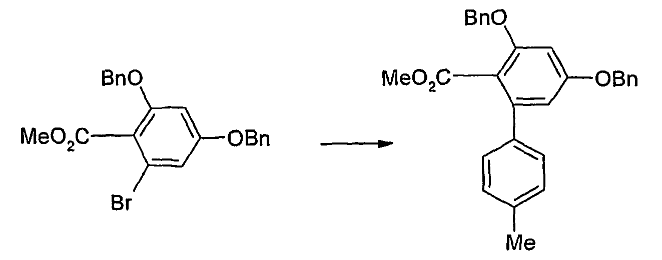 1,28 g 2,4-Bis-benzyloxy-6-brom-benzoesäure-methylester, 540 mg p-Tolylboronsäure, 1,2 g Natriumtetraborat·10 H₂O, 42,1 mg Bis-(triphenylphosphin)-palladium(II)-chlorid und 0,01 ml NH₂NH₂·H₂O werden mit 10 ml THF und 5 ml H₂O 6 h unter Rückfluß erhitzt. Es werden nochmals 200 mg Boronsäure und 400 mg Natriumtetraborat·10 H₂O zugegeben und weitere 4 h erhitzt. Das THF wird abgezogen, der Rückstand mit H₂O verdünnt und 3x mit EtOAc extrahiert. Die vereinigten organischen Extrakte werden getrocknet, eingeengt und über Kieselgel chromatographiert. Die sauberen Fraktionen kristallisieren im Eisschrank über (Me₂CH)₂O. Ausbeute: 300 mg 3,5-Bis-benzyloxy-4'-methyl-biphenyl-2-carbonsäure-methylester (23 %); F. 120°.
2.4 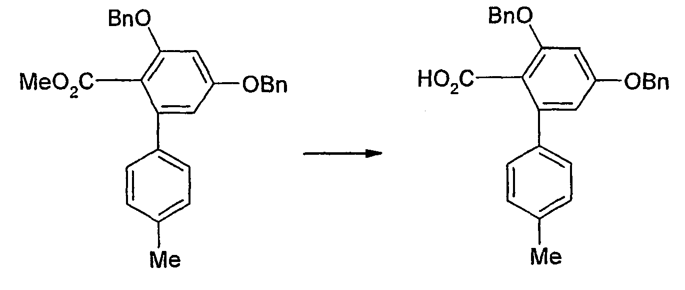 In einer geschlossenen Glasbombe werden 820 mg 3,5-Bis-benzyloxy-4'-methyl-biphenyl-2-carbonsäure-methylester, 1,5 ml 32 %ige NaOH und 7 ml Me₂CHOH 2 h bei 135 °C gerührt. Die Lösung wird eingeengt, mit H₂O verdünnt, mit HCl angesäuert und 3x mit EtOAc extrahiert. Die vereinigten organischen Extrakte werden getrocknet, eingeengt und mit (Me₂CH)₂O verrieben. Ausbeute: 450 mg 3,5-Bis-benzyloxy-4'-methyl-biphenyl-2-carbonsäure (57 %); F. 307-310°.
2.5 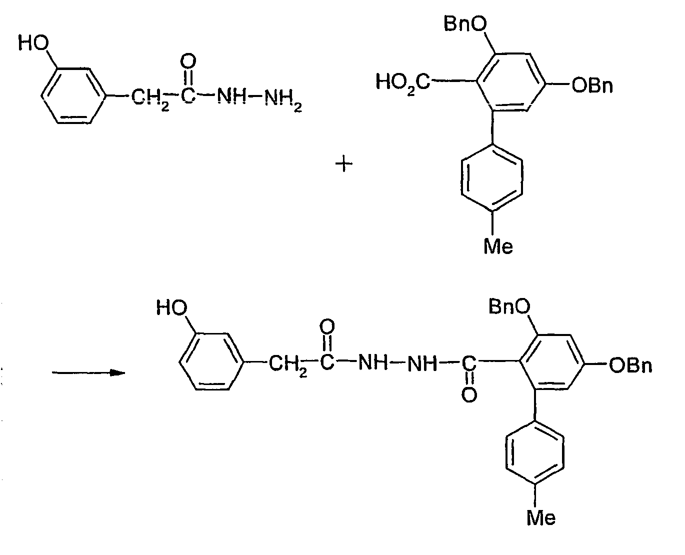 295 mg 3,5-Bis-benzyloxy-4'-methyl-biphenyl-2-carbonsäure, 190 mg 1-Ethyl-3-(3-dimethylaminopropyl)-carbodiimid (WSCD) und 110 mg 1-Hydroxybenzotriazol (HOBt) werden 4 h bei 36 °C in 1,5 ml DMF gerührt. 200 mg (3-Hydroxy-phenyl)-essigsäure-hydrazid werden zugefügt und es wird über Nacht bei 36 °C weiter gerührt. Das Reaktionsgemisch wird mit 2 ml MeOH verdünnt, in H₂O eingerührt und der Niederschlag abgesaugt. Der Feststoff wird in 20 ml EtOAc gelöst, getrocknet und aus Me₂CHOH/Et₂O kristallisiert. Ausbeute: 240 mg 3,5-Bis-benzyloxy-4'-methyl-biphenyl-2-carbonsäure-*N'*-[2-(3-hydroxy-phenyl)-acetyl]-hydrazid (60 %); F. 168-169°.
2.6 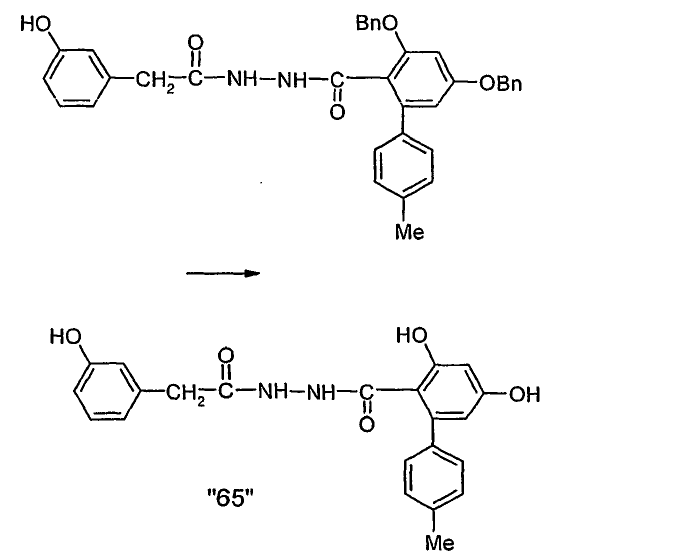 3,5-Bis-benzyloxy-4'-methyl-biphenyl-2-carbonsäure-N'-[2-(3-hydroxyphenyl)-acetyl]-hydrazid wird analog Beispiel 1.3 hydriert. Man erhält "65" (22 %); F. 97° (Zersetzung).

### Beispiel 3

Herstellung von 2,4,6-Trihydroxy-benzoesäure-N'-[2-(3-hydroxy-phenyl)-acetyl]-hydrazid ("68")
3.1 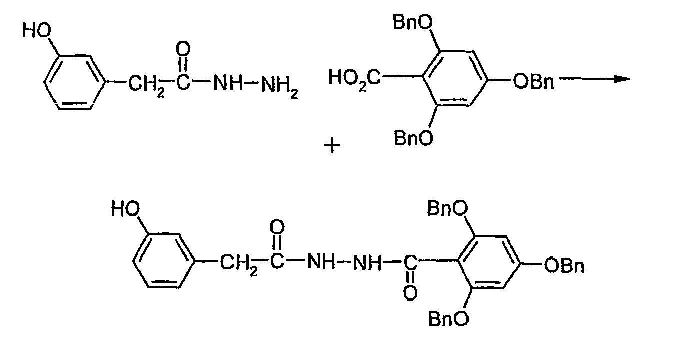 Die Kupplung von 2,4,6-Tribenzyloxy-benzoesäure mit (3-Hydroxy-phenyl)-essigsäure-hydrazid wird analog Beispiel 2.5 durchgeführt. Man erhält 2,4,6-Tribenzyloxy-benzoesäure-*N'*-[2-(3-hydroxy-phenyl)-acetyl]-hydrazid, Ausbeute 40 %; F. 154-155°.
3.2 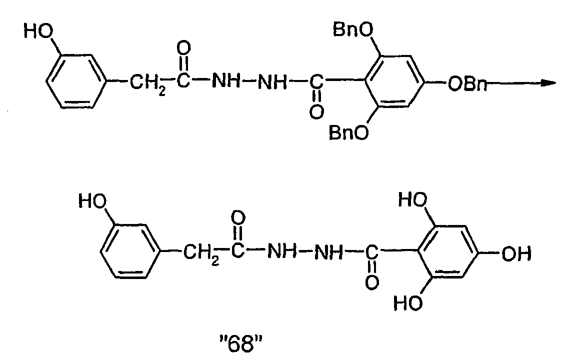 2,4,6-Tribenzyloxy-benzoesäure-N'-[2-(3-hydroxy-phenyl)-acetyl]-hydrazid wird analog Beispiel 1.3 hydriert. Man erhält "68" (Ausbeute 81 %); F. 237-238°.

### Beispiel 4

Herstellung von 2,4-Dihydroxy-6-methyl-benzoesäure-*N*'-[2-(3,5-dihydroxyphenyl)-acetyl]-hydrazid ("67")
4.1 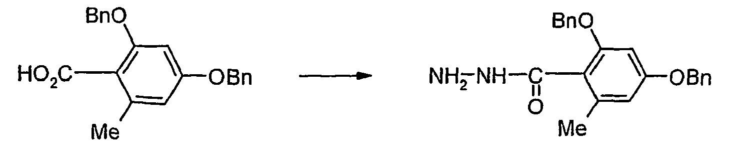 N₂H₅OH wird analog Beispiel 2.5 mit 2,4-Dibenzyloxy-6-methyl-benzoesäure monoacyliert. Ausbeute: 2,4-Dibenzyloxy-6-methyl-benzoesäure-hydrazid (63 %); F. 136-137°.
4.2 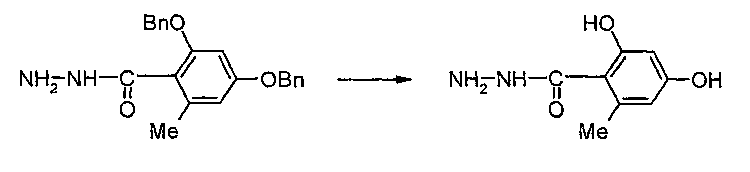 2,4-Dibenzyloxy-6-methyl-benzoesäure-hydrazid wird analog Beispiel 1.3 hydriert. Ausbeute: 2,4-Dihydroxy-6-methyl-benzoesäure-hydrazid (89 %); F. 226° (Zersetzung).
4.3 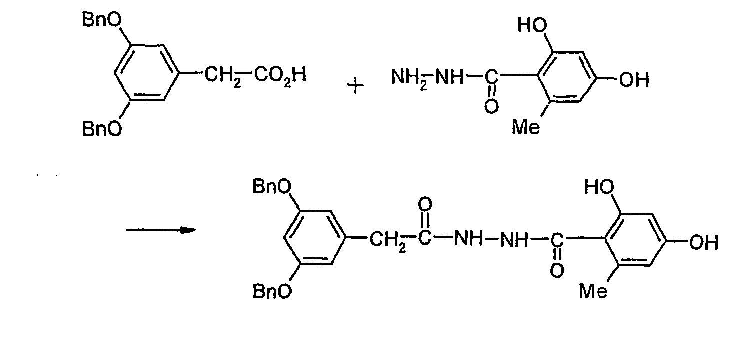 Die Kupplung von (3,5-Bis-benzyloxy-phenyl)-essigsäure mit 2,4-Dihydroxy-6-methyl-benzoesäure-hydrazid wird analog Beispiel 2.5 durchgeführt. Ausbeute: 2,4-Dihydroxy-6-methyl-benzoesäure-N'-[2-(3,5-dibenzyloxy-phenyl)-acetyl]-hydrazid (39 %).
4.4 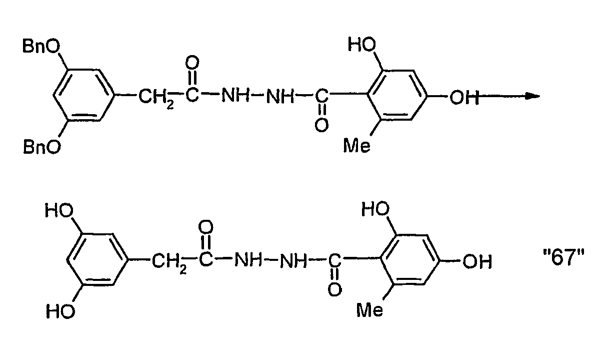 2,4-Dihydroxy-6-methyl-benzoesäure-N'-[2-(3,5-dibenzyloxy-phenyl)-acetyl]-hydrazid wird analog Beispiel 1.3 hydriert. Ausbeute: "67" (83 %); F. 281° (Zersetzung).

### Beispiel 5

5.1 Analog Beispiel 2.5 erhält man 2-Chlor-4,6-dimethoxy-benzoesäure-*N*'-[2-(3-hydroxy-phenyl)-acetyl]-hydrazid ("69a").
5.2 9 g "69a" werden in 30 ml Dichlormethan suspendiert. Unter Eiskühlung werden 40 ml BBr₃ zugetropft. Nach 48 Stunden bei Raumtemperatur rührt man 200 ml Eiswasser ein. Man arbeitet wie üblich auf, trennt über Kieselgel mittels eines CombiFlash COMPANION Gerätes und erhält nach Kristallisation aus Diethylether 3,3 g man 2-Chlor-4,6-dihydroxy-benzoesäure-*N*'-[2-(3-hydroxy-phenyl)-acetyl]-hydrazid ("70"), F. 217°

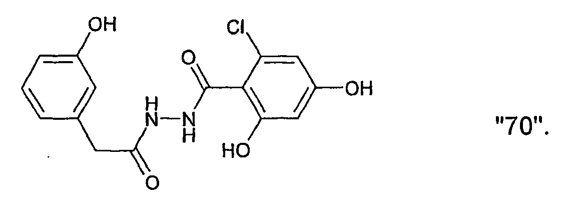

### Beispiel 6

6.1 Analog Beispiel 2.5 und 2.6 erhält man 4-Hydroxy-3-nitrobenzoesäure-*N*'-[2-(3-hydroxy-phenyl)-acetyl]-hydrazid ("74"), F. 190-193°.
6.2 "74" wird nach Standardverfahren mit Pd/C in THF hydriert. Der Katalysator und das Lösungsmittel werden abgetrennt. Der Rückstand wird mit etwas Methanol/HCl versetzt. Der Niederschlag wird abgetrennt und getrocknet. Man erhält 3-Amino-4-hydroxy-benzoesäure-*N*'-[2-(3-hydroxyphenyl)-acetyl]-hydrazid ("78"), Ausbeute 79 %, F. 264-265°.

Analog erhält man die nachstehenden Verbindungen

| Nr. | Strukturformel | F. [°C] |
|---|---|---|
| 1 | 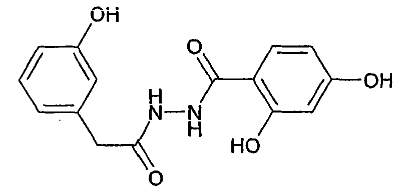 | 233-235 |
| 2 | 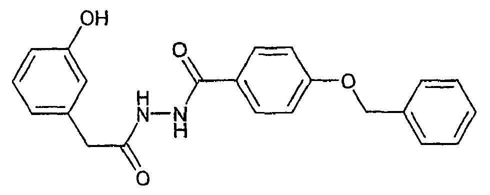 | 199-200 |
| 3 | 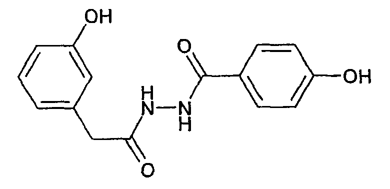 | 207-208 |
| 4 | 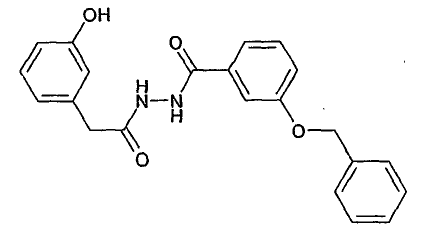 | 152-153 |
| 5 | 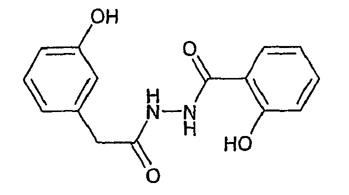 | 214-215 |
| 6 | 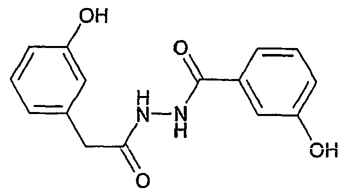 | 224-225 |
| 7 | 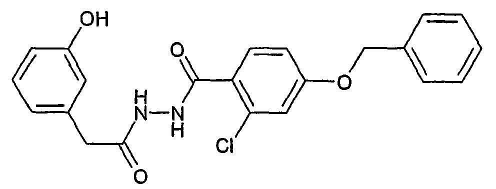 | 162-164 |
| 8 | 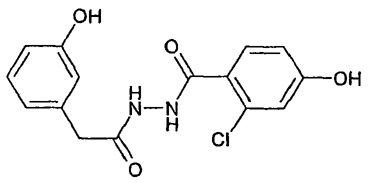 | 165-166 |
| 9 | 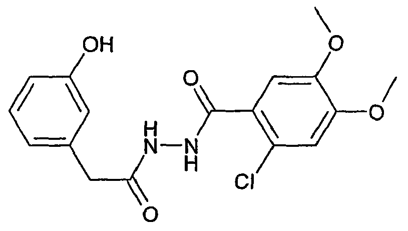 | 174-175 |
| 10 | 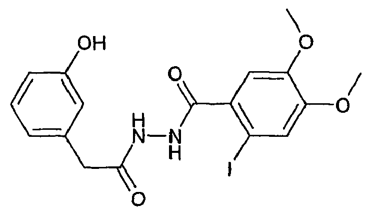 | 199-200 |
| 11 | 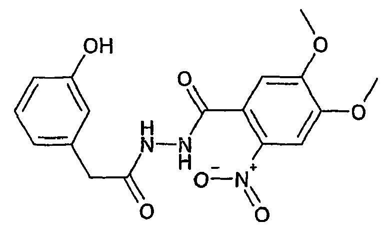 | 226-227 |
| 12 | 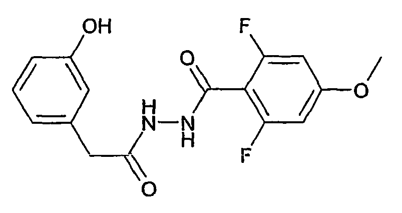 | 198-199 |
| 13 | 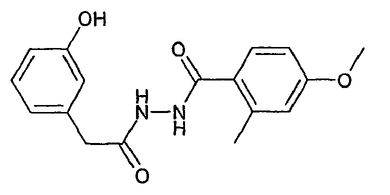 | 171-172 |
| 14 | 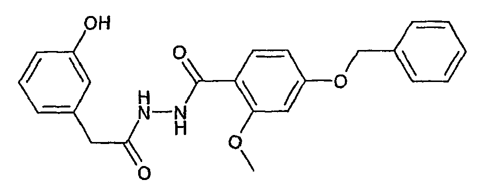 | 193-194 |
| 15 | 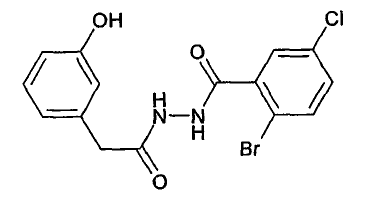 | 212-213 |
| 16 | 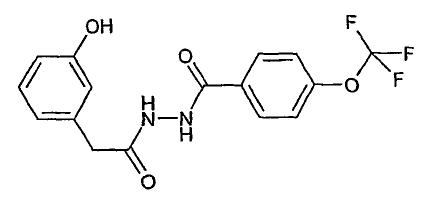 | 189-190 |
| 17 | 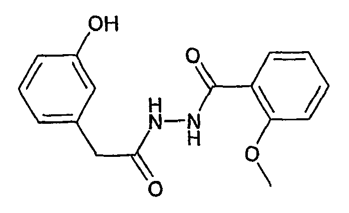 | 181-182 |
| 18 | 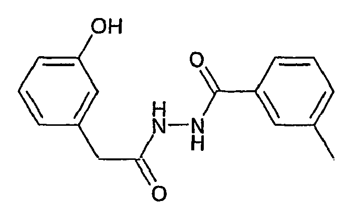 | 156-157 |
| 19 | 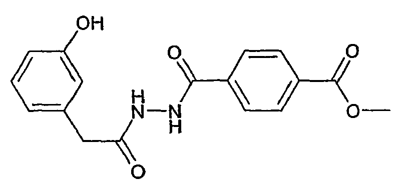 | 201-202 |
| 20 | 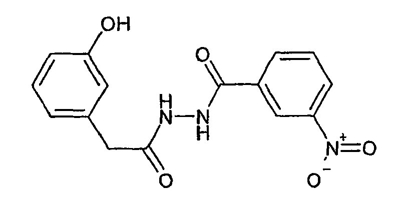 | 179 |
| 21 | 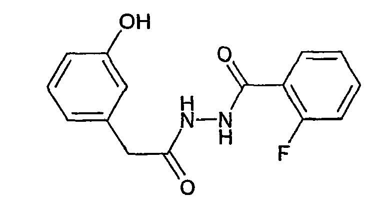 | 154-155 |
| 22 | 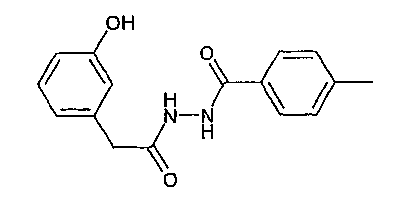 | 175-176 |
| 23 | 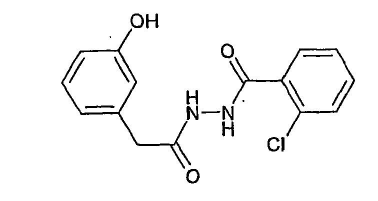 | 172 |
| 24 | 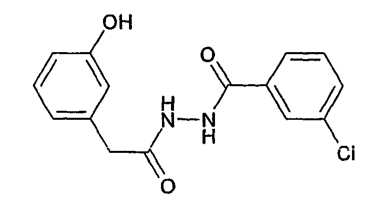 | 183-184 |
| 25 | 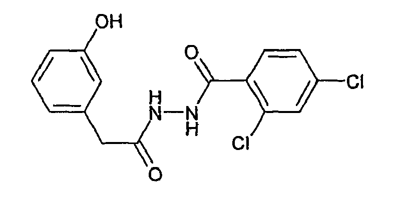 | 202-203 |
| 26 | 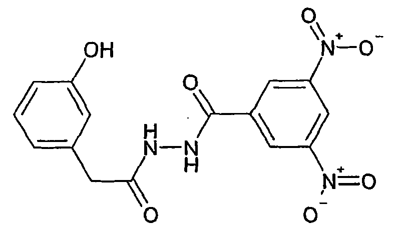 | 248-249 |
| 27 | 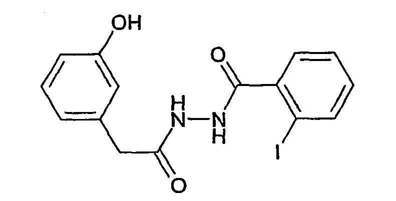 | 190-191 |
| 28 | 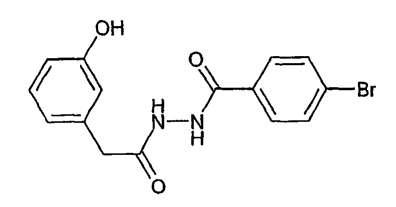 | 229 |
| 29 | 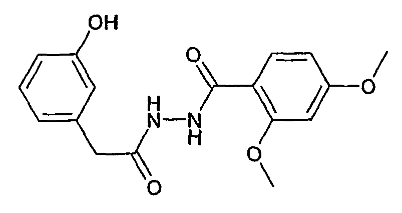 | 197-198 |
| 30 | 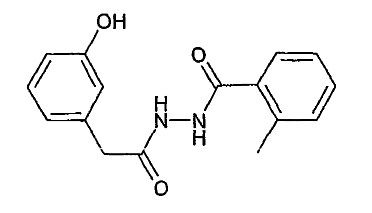 | 197-198 |
| 31 | 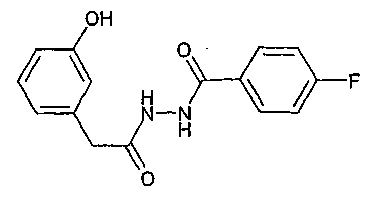 | 182-183 |
| 32 | 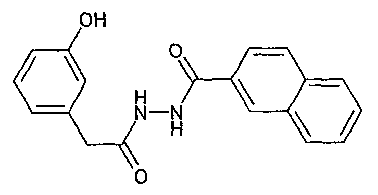 | 201-202 |
| 33 | 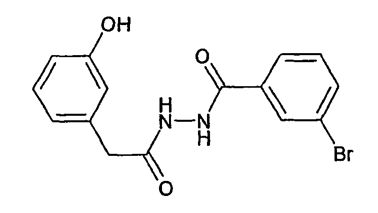 | 194-195 |
| 34 | 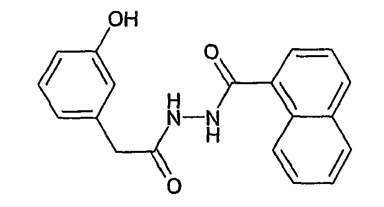 | 210-211 |
| 35 | 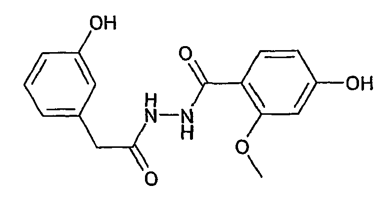 | 208-209 |
| 36 | 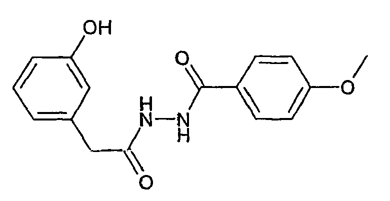 | 158-159 |
| 37 | 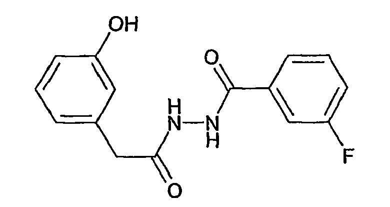 | 176-177 |
| 38 | 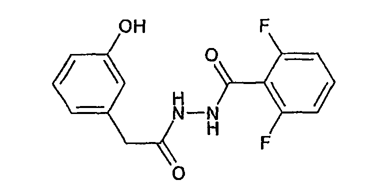 | 191-192 |
| 39 | 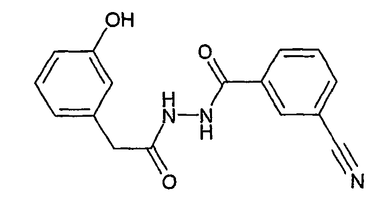 | 181.5-182.5 |
| 40 | 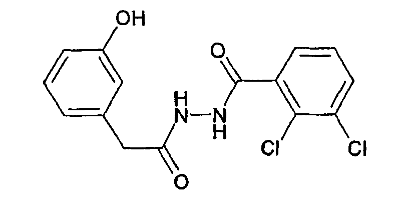 | 220-221 |
| 41 | 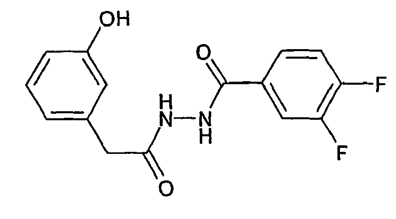 | 164 |
| 42 | 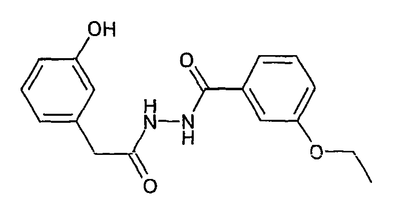 | 130-132 |
| 43 | 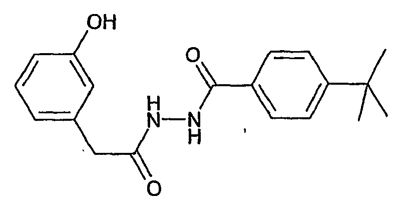 | 183-184 |
| 44 | 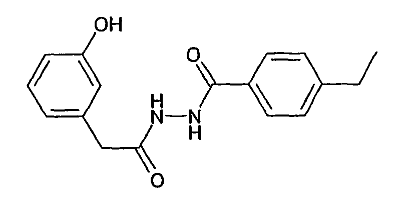 | 155-156 |
| 45 | 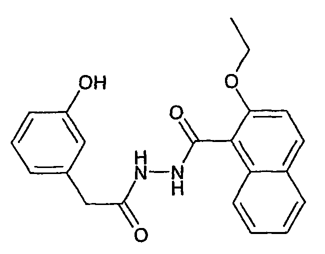 | 148-150 |
| 46 | 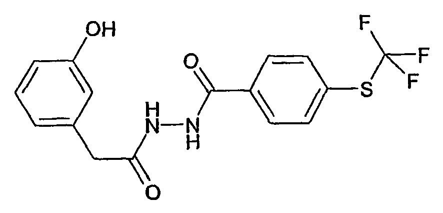 | 165-166 |
| 47 | 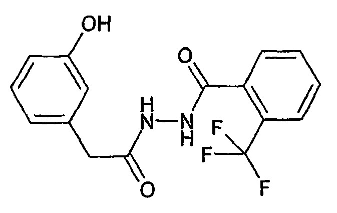 | 195-196 |
| 48 | 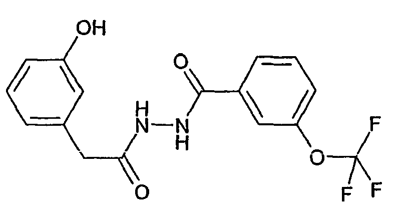 | 256-257 |
| 49 | 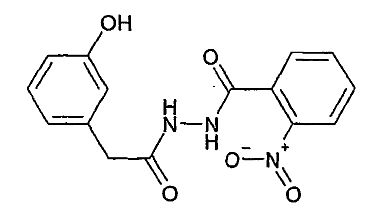 | 206-207 |
| 50 | 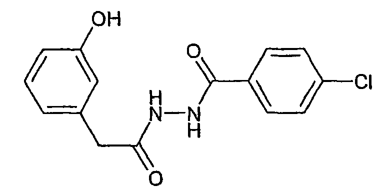 | 223 |
| 51 | 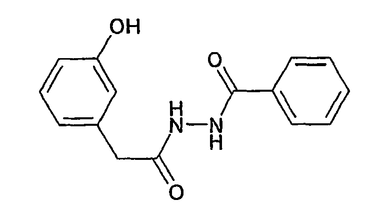 | 162-163 |
| 52 | 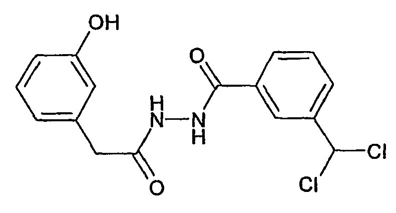 | 154-155 |
| 53 | 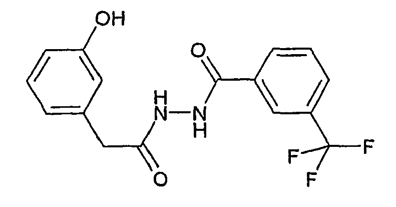 | 161-162 |
| 54 | 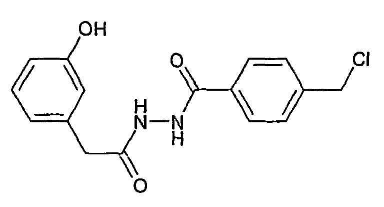 | 175-176 |
| 55 | 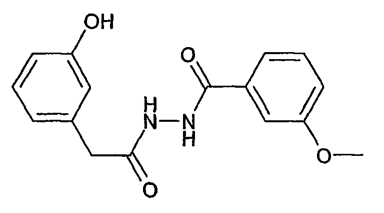 | 138-139 |
| 56 | 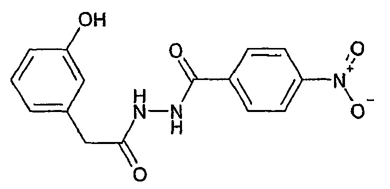 | 222-223 |
| 57 | 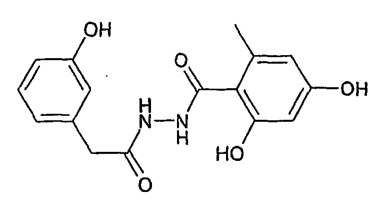 | 226-227 |
| 58 | 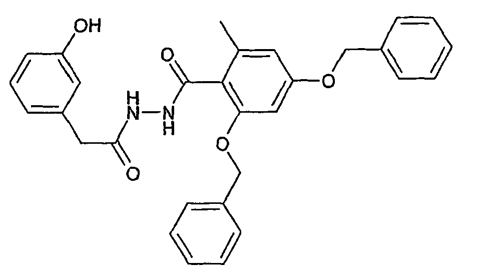 | 165-166 |
| 61 | 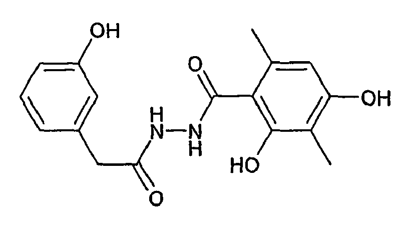 | 193-194 |
| 62 | 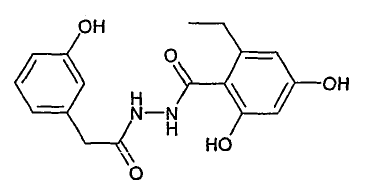 | 251 |
| 63 | 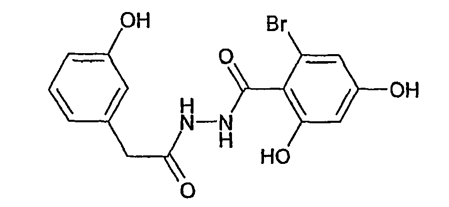 | 226-227 |
| 64 | 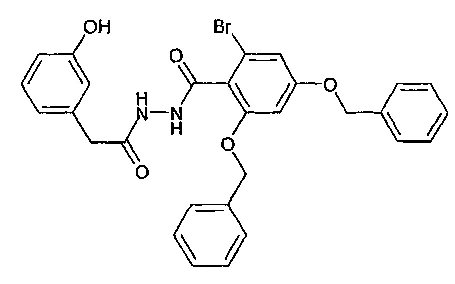 | 194-195 |
| 66 | 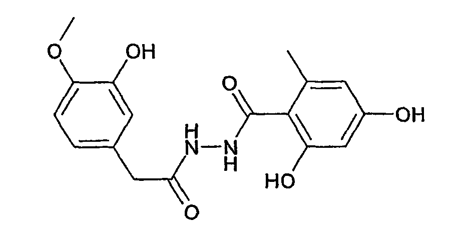 | 232-233 |
| 69 | 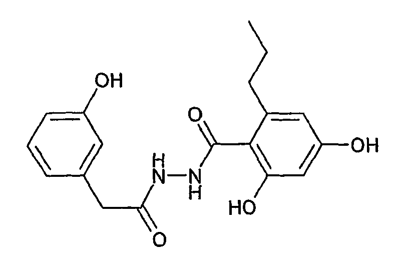 | 198-200 |
| 71 | 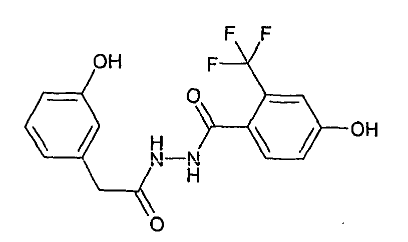 | 210-212 |
| 72 | 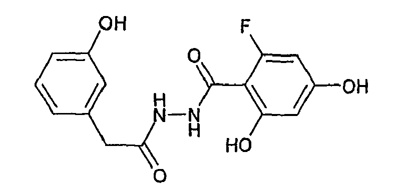 | 230-232 |
| 73 | 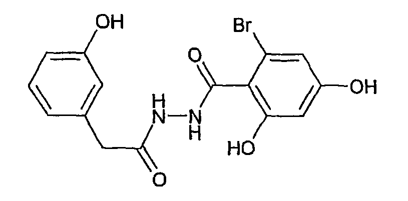 | 259 |
| 75 | 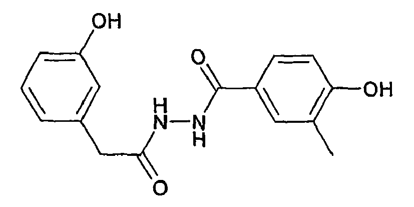 | 207-208 |
| 76 | 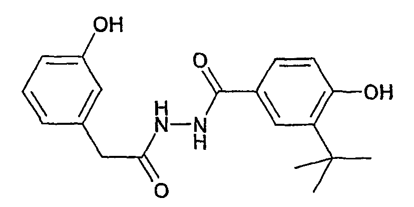 | 154-157 |
| 77 | 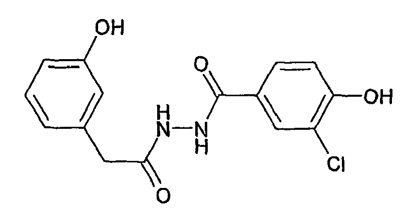 | 230-231 |
| 79 | 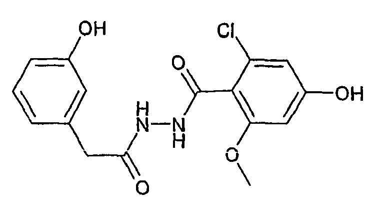 | 105-106 |
| 80 | 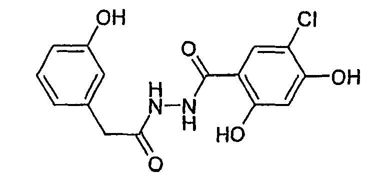 | 269-270 |
| 81 | 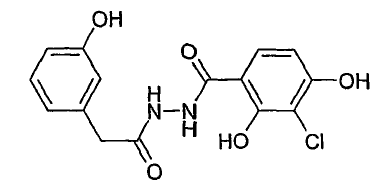 | 252-253 |
| 82 | 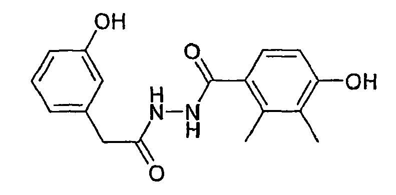 | 251 |
| 83 | 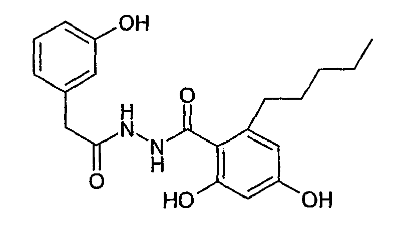 | 230 |
| 84 | 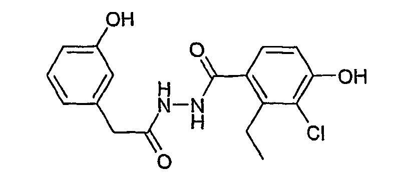 | 259 |
| 85 | 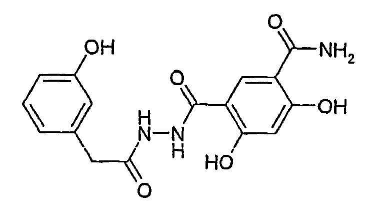 | 260-261 |
| 86 | 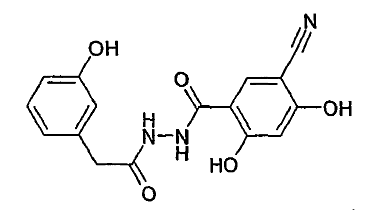 | 258 |
| 87 | 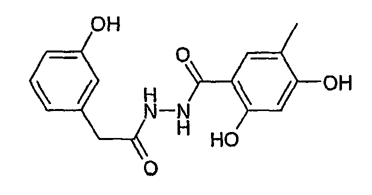 | 240 |
| 88 | 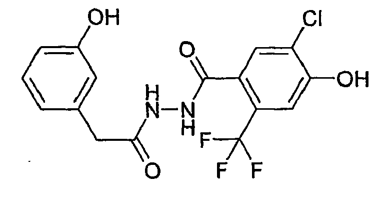 | 200-202 |

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 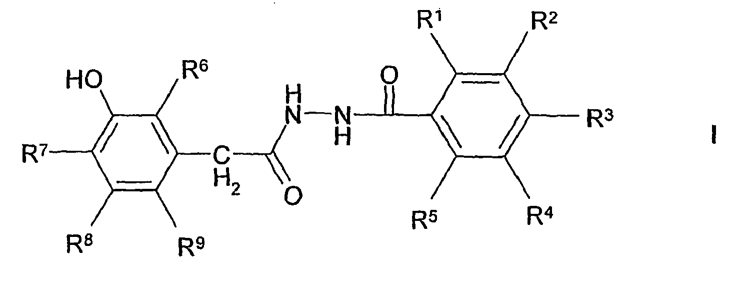 worin
R¹, R², R³, R⁴, R⁵,
R⁶, R⁷, R⁸, R⁹ jeweils unabhängig voneinander H, A, OSO₂A, Hal, NO₂, OR¹⁰, N(R¹⁰)₂, CN, -[C(R¹⁰)₂]ₙCOOR¹⁰, O-[C(R¹⁰)₂]ₒCOOR¹⁰, SO₃H, -[C(R¹⁰)₂]ₙAr, -CO-Ar, O-[C(R¹⁰)₂]ₙAr, -[C(R¹⁰)₂]ₙHet, -[C(R¹⁰)₂]ₙC≡CH, O-[C(R¹⁰)₂]ₙC≡CH, -[C(R¹⁰)₂]ₙCON(R¹⁰)₂, -[C(R¹⁰)₂]ₙCONR¹⁰N(R¹⁰)₂, O-[C(R¹⁰)₂]ₙCON(R¹⁰)₂, O-[C(R¹⁰)₂]ₒCONR¹⁰N(R¹⁰)₂, NR¹⁰COA, NR¹⁰CON(R¹⁰)₂, NR¹⁰SO₂A, N(SO₂A)₂, COR¹⁰, S(O)ₘAr, SO₂NR¹⁰ oder S(O)ₘA,
R¹ und R², R² und R³,
R³ und R⁴ oder R⁴ und R⁵ zusammen auch CH=CH-CH=CH,
A unverzweigtes oder verzweigtes Alkyl mit 1-6 C-Atomen, worin 1-7 H-Atome durch F ersetzt sein können, oder cylisches Alkyl mit 3-7 C-Atomen,
Ar unsubstituiertes oder ein-, zwei- oder dreifach durch Hal, A, OR¹⁰, N(R¹⁰)₂, NO₂, CN, Phenyl, CON(R¹⁰)₂, NR¹⁰COA, NR¹⁰CON(R¹⁰)₂, NR¹⁰SO₂A, COR¹⁰, SO₂N(R¹⁰)₂, S(O)ₘA, -[C(R¹⁰)₂]ₙ-COOR¹⁰ und/oder -O[C(R¹⁰)₂]ₒ-COOR¹⁰ substituiertes Phenyl, Naphthyl oder Biphenyl,
Het einen ein- oder zweikernigen gesättigten, ungesättigten oder aromatischen Heterocyclus mit 1 bis 4 N-, O- und/oder S-Atomen, der ein-, zwei- oder dreifach durch Hal, A, OR¹⁰, N(R¹⁰)₂, NO₂, CN, COOR¹⁰, CON(R¹⁰)₂, NR¹⁰COA, NR¹⁰SO₂A, COR¹⁰, SO₂NR¹⁰, S(O)ₘA, =S, =NR¹⁰ und/oder =O (Carbonylsauerstoff) substituiert sein kann,
R¹⁰ H oder A,
Hal F, Cl, Br oder I,
m 0, 1 oder 2,
n 0, 1, 2 oder 3,
o 1, 2 oder 3
bedeuten,
sowie ihre pharmazeutisch verwendbaren Derivate, Salze, Solvate und Stereoisomere, einschließlich deren Mischungen in allen Verhältnissen.

2. Verbindungen nach Anspruch 1, worin
R¹ H, A, Hal, NO₂, OR¹⁰, -[C(R¹⁰)₂]ₙAr oder O-[C(R¹⁰)₂]ₙAr
bedeutet,
sowie ihre pharmazeutisch verwendbaren Derivate, Salze, Solvate und Stereoisomere, einschließlich deren Mischungen in allen Verhältnissen.

3. Verbindungen nach Anspruch 1 oder 2, worin
R² H, A, Hal, CN, NO₂, OR¹⁰, -[C(R¹⁰)₂]ₙAr oder O-[C(R¹⁰)₂]ₙAr
bedeutet,
sowie ihre pharmazeutisch verwendbaren Derivate, Salze, Solvate und Stereoisomere, einschließlich deren Mischungen in allen Verhältnissen.

4. Verbindungen nach einem oder mehreren der Ansprüche 1-3, worin R³ H, A, Hal, NO₂, OR¹⁰, -[C(R¹⁰)₂]ₙAr, O-[C(R¹⁰)₂]ₙAr, -[C(R¹⁰)₂]ₙCOOR¹⁰ oder S(O)ₘA
bedeutet,
sowie ihre pharmazeutisch verwendbaren Derivate, Salze, Soivate und Stereoisomere, einschließlich deren Mischungen in allen Verhältnissen.

5. Verbindungen nach einem oder mehreren der Ansprüche 1-4, worin R⁴ H, A, Hal, CONH₂, CN, NO₂ oder OR¹⁰
bedeutet,
sowie ihre pharmazeutisch verwendbaren Derivate, Salze, Solvate und Stereoisomere, einschließlich deren Mischungen in allen Verhältnissen.

6. Verbindungen nach einem oder mehreren der Ansprüche 1-5, worin R⁵ H, A, Hal, OR¹⁰, -[C(R¹⁰)₂]ₙAr oder O-[C(R¹⁰)₂]ₙAr
bedeutet,
sowie ihre pharmazeutisch verwendbaren Derivate, Salze, Solvate und Stereoisomere, einschließlich deren Mischungen in allen Verhältnissen.

7. Verbindungen nach einem oder mehreren der Ansprüche 1-6, worin R⁶ H oder A bedeutet,
sowie ihre pharmazeutisch verwendbaren Derivate, Salze, Solvate und Stereoisomere, einschließlich deren Mischungen in allen Verhältnissen.

8. Verbindungen nach einem oder mehreren der Ansprüche 1-7, worin R⁷ H, A oder OR¹⁰ bedeutet,
sowie ihre pharmazeutisch verwendbaren Derivate, Salze, Solvate und Stereoisomere, einschließlich deren Mischungen in allen
Verhältnissen.

9. Verbindungen nach einem oder mehreren der Ansprüche 1-8, worin R⁸ H, A oder OR¹⁰ bedeutet,
sowie ihre pharmazeutisch verwendbaren Derivate, Salze, Solvate und Stereoisomere, einschließlich deren Mischungen in allen Verhältnissen.

10. Verbindungen nach einem oder mehreren der Ansprüche 1-9, worin R⁹ H oder A bedeutet,
sowie ihre pharmazeutisch verwendbaren Derivate, Salze, Solvate und Stereoisomere, einschließlich deren Mischungen in allen Verhältnissen.

11. Verbindungen nach einem oder mehreren der Ansprüche 1-10, worin
Ar unsubstituiertes oder ein-, zwei- oder dreifach durch Hal und/oder A substituiertes Phenyl bedeutet,
sowie ihre pharmazeutisch verwendbaren Derivate, Salze, Solvate und Stereoisomere, einschließlich deren Mischungen in allen Verhältnissen.

12. Verbindungen nach einem oder mehreren der Ansprüche 1-11, worin
Het einen einkernigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann,
bedeutet,
sowie ihre pharmazeutisch verwendbaren Derivate, Salze, Solvate und Stereoisomere, einschließlich deren Mischungen in allen Verhältnissen.

13. Verbindungen nach einem oder mehreren der Ansprüche 1-12, worin
Het einen einkernigen gesättigten Heterocyclus mit 1 bis 2 N- und/oder O-Atomen, der unsubstituiert oder ein- oder zweifach durch A substituiert sein kann,
bedeutet,
sowie ihre pharmazeutisch verwendbaren Derivate, Salze, Solvate und Stereoisomere, einschließlich deren Mischungen in allen Verhältnissen.

14. Verbindungen nach einem oder mehreren der Ansprüche 1-13, worin
Het unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl,
bedeutet,
sowie ihre pharmazeutisch verwendbaren Derivate, Salze, Solvate und Stereoisomere, einschließlich deren Mischungen in allen Verhältnissen.

15. Verbindungen nach einem oder mehreren der Ansprüche 1-14, worin R¹ H, A, Hal, NO₂, OR¹⁰, -[C(R¹⁰)₂]ₙAr oder O-[C(R¹⁰)₂]ₙAr,
R² H, A, Hal, CN, N(R¹⁰)₂, NO₂, OR¹⁰, -[C(R¹⁰)₂]ₙAr oder O-[C(R¹⁰)₂]ₙAr,
R³ H, A, Hal, NO₂, OR¹⁰, -[C(R¹⁰)₂]ₙAr, O-[C(R¹⁰)₂]ₙAr. -[C(R¹⁰)₂]ₙCOOR¹⁰ oder S(O)ₘA,
R⁴ H, A, Hal, CONH₂, CN, NO₂ oder OR¹⁰,
R⁵ H, A, Hal, OR¹⁰, -[C(R¹⁰)₂]ₙAr oder O-[C(R¹⁰)₂]ₙAr,
R⁶ H,
R⁷ H oder OR¹⁰,
R⁸ H oder OR¹⁰,
R⁹ H,
R¹ und R², R² und R³,
R³ und R⁴ oder R⁴ und R⁵ zusammen auch CH=CH-CH=CH,
A unverzweigtes oder verzweigtes Alkyl mit 1-6 C-Atomen, worin 1-7 H-Atome durch F ersetzt sein können,
Ar unsubstituiertes oder ein-, zwei- oder dreifach durch Hal und/oder A substituiertes Phenyl,
R¹⁰ H oder A,
Hal F, Cl, Br oder I,
m 0, 1 oder 2,
n 0, 1, 2 oder 3
bedeuten,
sowie ihre pharmazeutisch verwendbaren Derivate, Salze, Solvate und Stereoisomere, einschließlich deren Mischungen in allen Verhältnissen.

16. Verbindungen nach einem oder mehreren der Ansprüche 1-15, worin R¹ OH, A oder Hal,
R² H, A oder Hal,
R³ OH,
R⁴ H, A oder Hal,
R⁵ H oder OH,
R⁶ H,
R⁷ H,
R⁸ H,
R⁹ H,
R¹ und R², R² und R³,
R³ und R⁴ oder R⁴ und R⁵ zusammen auch CH=CH-CH=CH,
A unverzweigtes oder verzweigtes Alkyl mit 1-6 C-Atomen, worin 1-7 H-Atome durch F ersetzt sein können,
Hal F, Cl, Br oder I
bedeuten,
sowie ihre pharmazeutisch verwendbaren Derivate, Salze, Solvate und Stereoisomere, einschließlich deren Mischungen in allen Verhältnissen.

17. Verbindungen nach Anspruch 1 ausgewählt aus der Gruppe
| Nr. | Strukturformel |
|---|---|
| 1 | 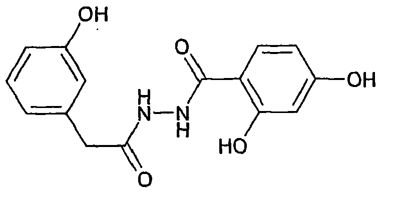 |
| 2 | 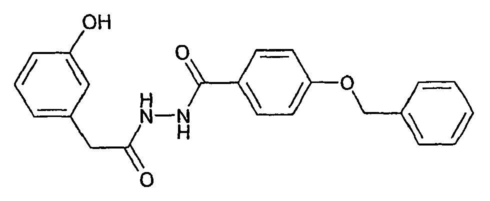 |
| 3 | 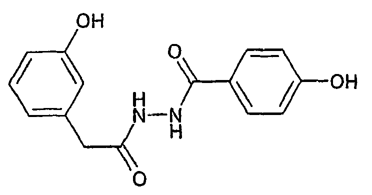 |
| 4 | 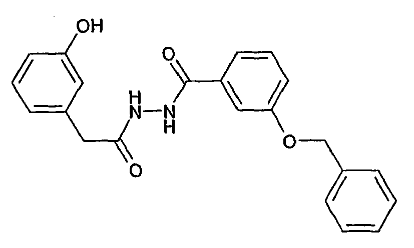 |
| 5 | 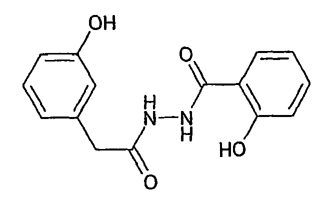 |
| 6 | 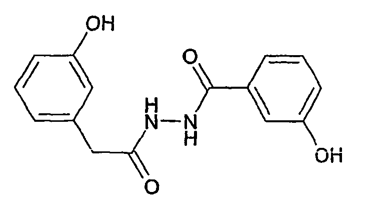 |
| 7 | 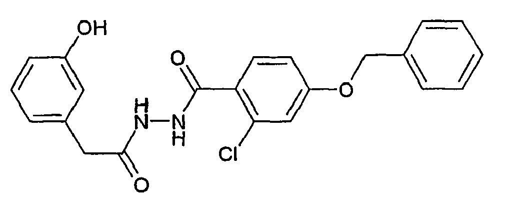 |
| 8 | 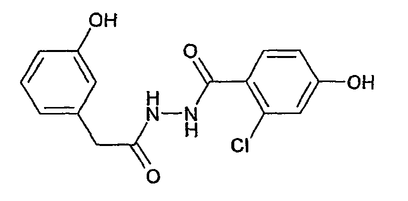 |
| 9 | 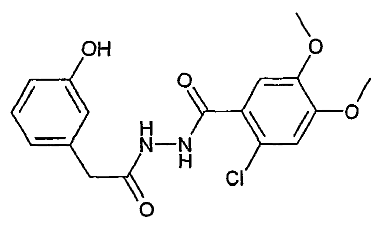 |
| 10 | 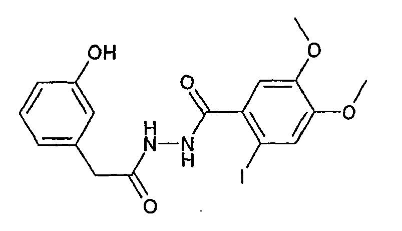 |
| 11 | 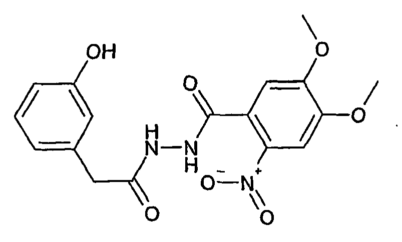 |
| 12 | 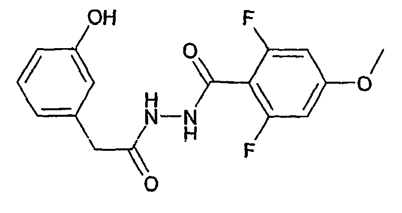 |
| 13 | 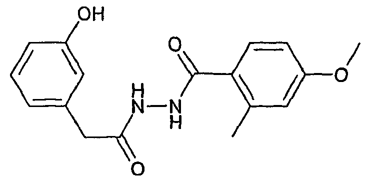 |
| 14 | 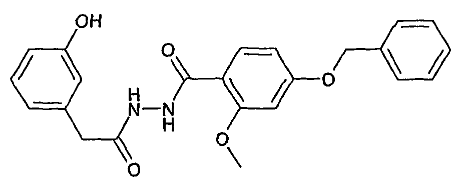 |
| 15 | 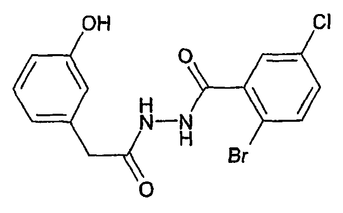 |
| 16 | 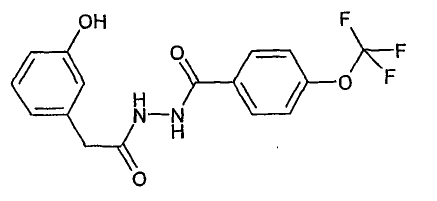 |
| 17 | 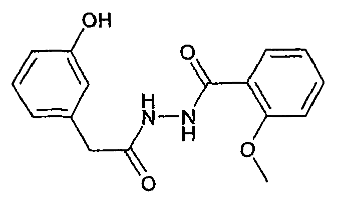 |
| 18 | 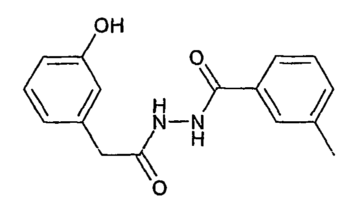 |
| 19 | 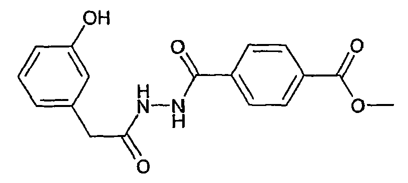 |
| 20 | 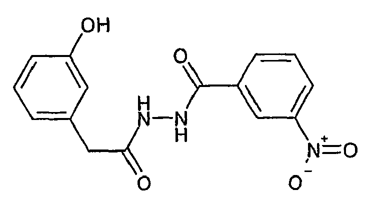 |
| 21 | 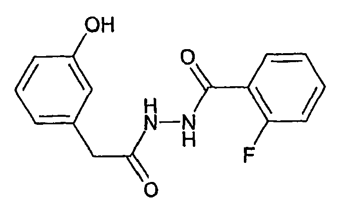 |
| 22 | 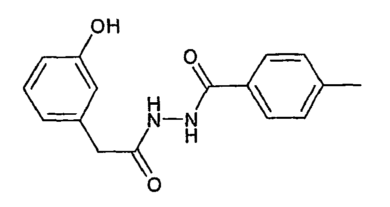 |
| 23 | 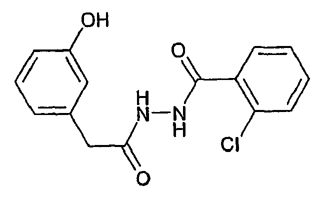 |
| 24 | 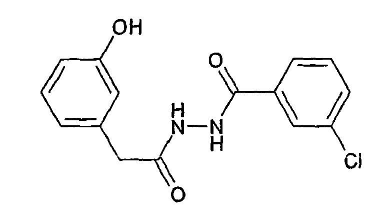 |
| 25 | 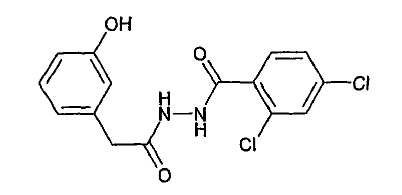 |
| 26 | 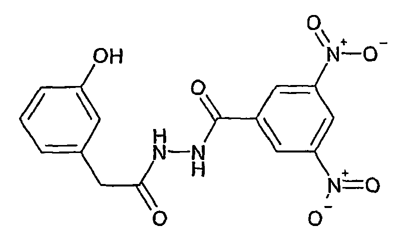 |
| 27 | 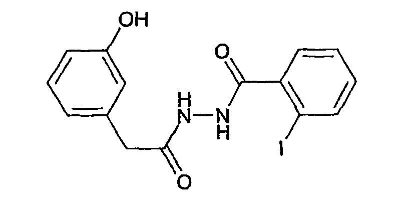 |
| 28 | 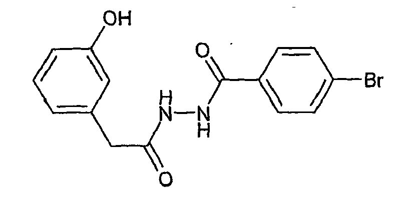 |
| 29 | 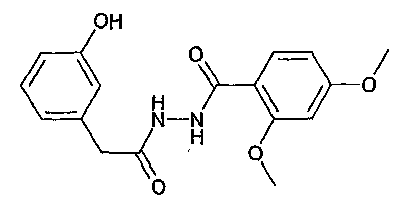 |
| 30 | 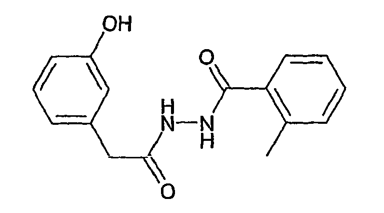 |
| 31 | 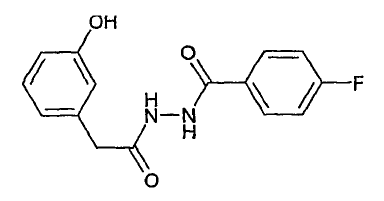 |
| 32 | 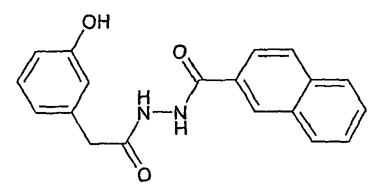 |
| 33 | 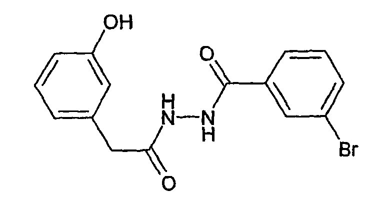 |
| 34 | 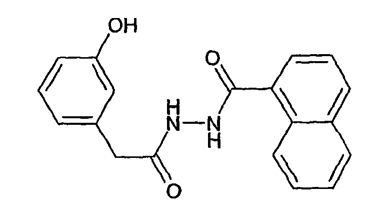 |
| 35 | 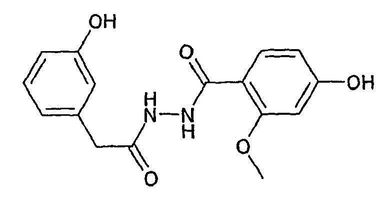 |
| 36 | 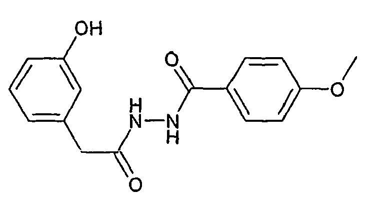 |
| 37 | 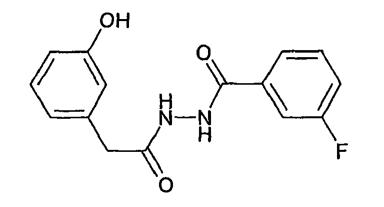 |
| 38 | 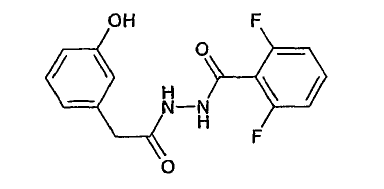 |
| 39 | 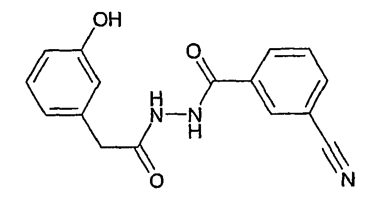 |
| 40 | 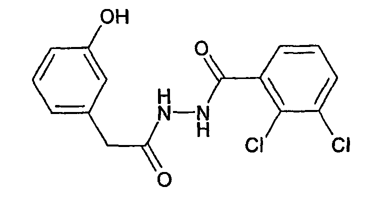 |
| 41 | 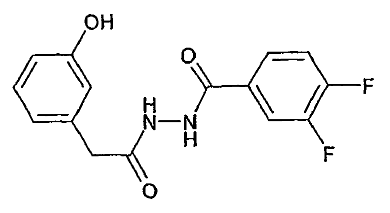 |
| 42 | 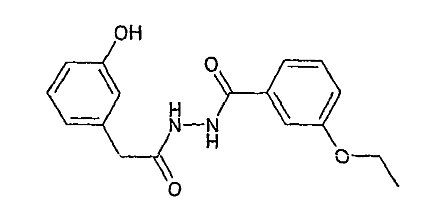 |
| 43 | 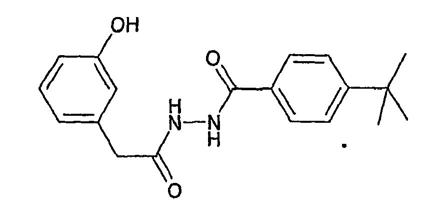 |
| 44 | 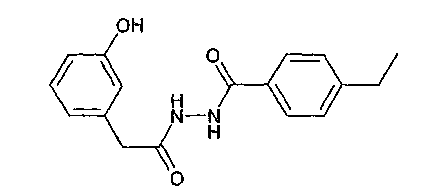 |
| 45 | 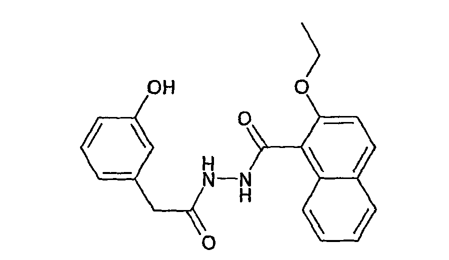 |
| 46 | 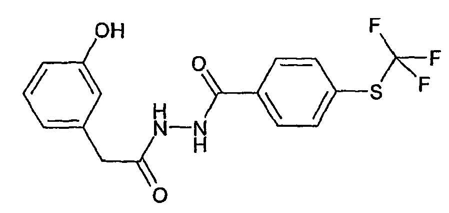 |
| 47 | 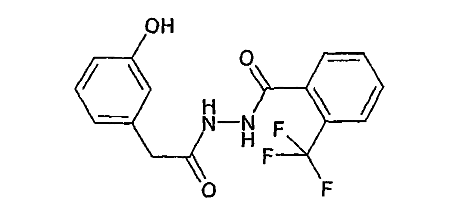 |
| 48 | 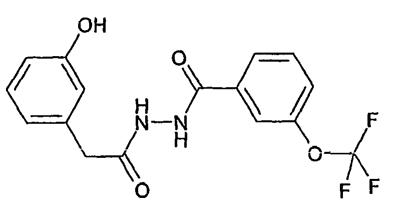 |
| 49 | 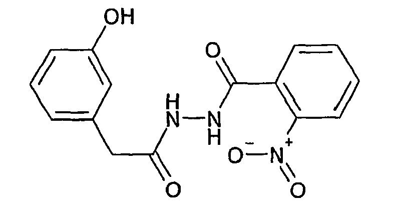 |
| 50 | 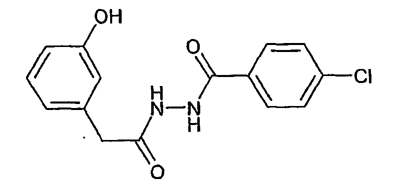 |
| 51 | 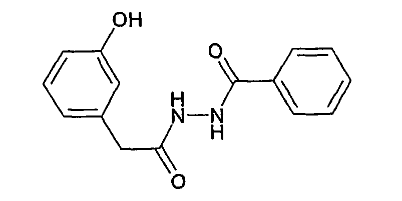 |
| 52 | 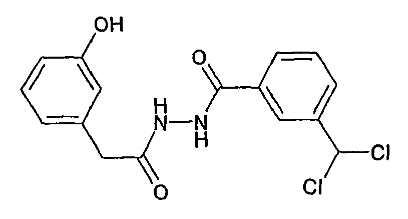 |
| 53 | 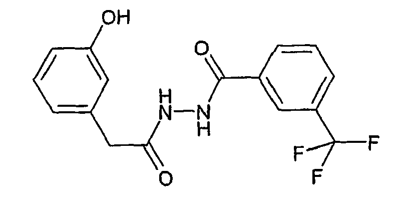 |
| 54 | 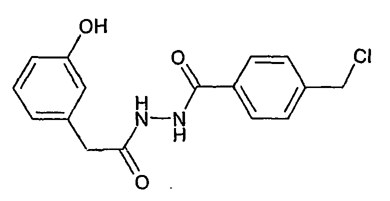 |
| 55 | 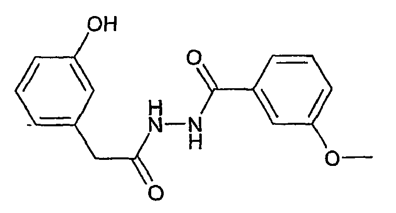 |
| 56 | 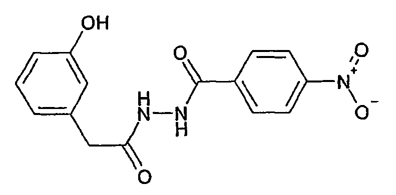 |
| 57 | 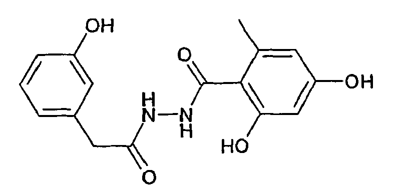 |
| 58 | 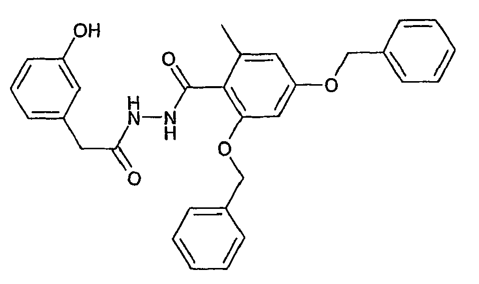 |
| 59 | 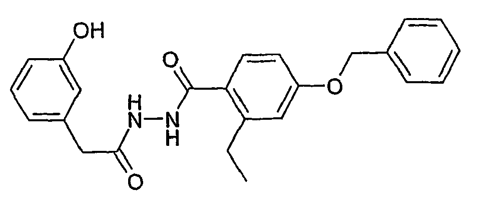 |
| 60 | 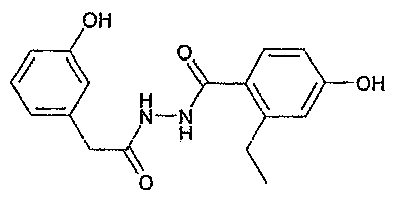 |
| 61 | 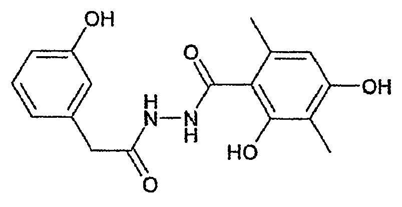 |
| 62 | 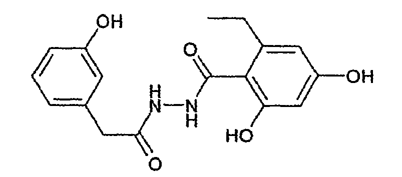 |
| 63 | 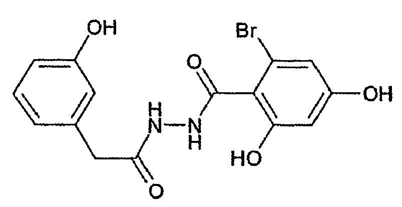 |
| 64 | 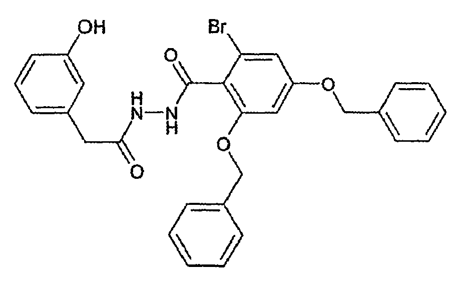 |
| 65 | 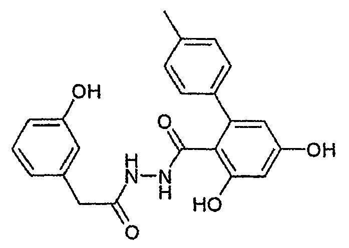 |
| 66 | 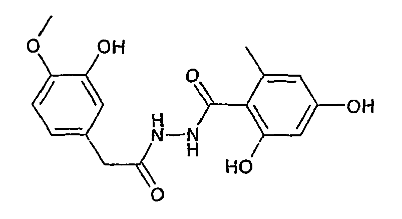 |
| 67 | 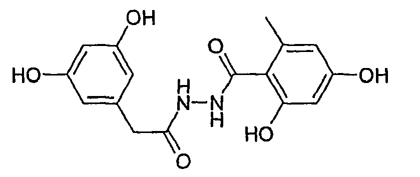 |
| 68 | 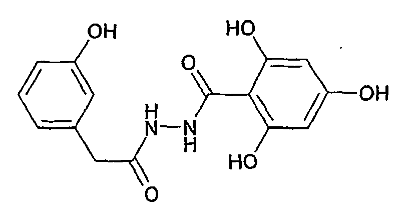 |
| 69 | 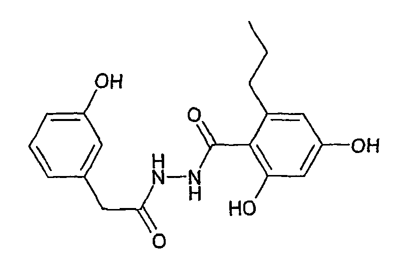 |
| 70 | 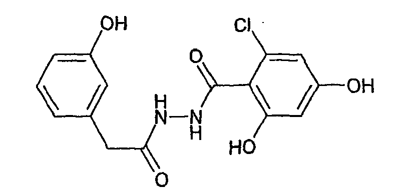 |
| 71 | 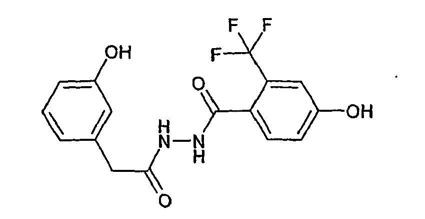 |
| 72 | 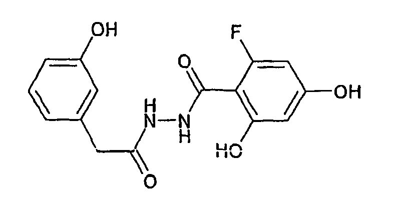 |
| 73 | 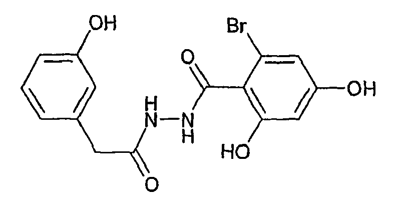 |
| 74 | 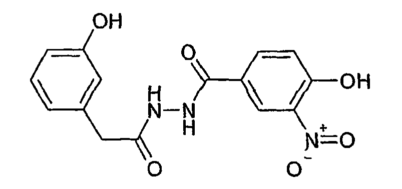 |
| 75 | 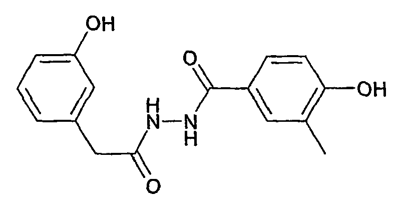 |
| 76 | 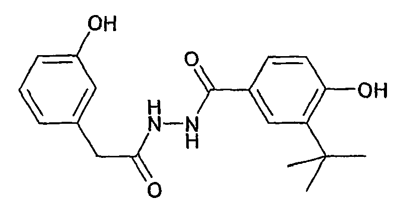 |
| 77 | 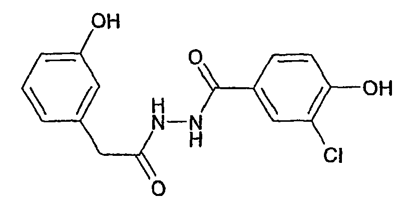 |
| 78 | 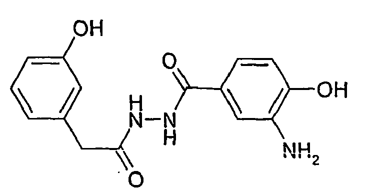 |
| 79 | 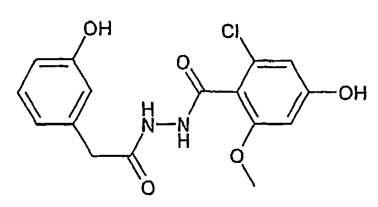 |
| 80 | 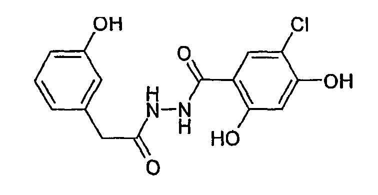 |
| 81 82 | 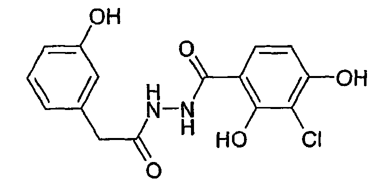 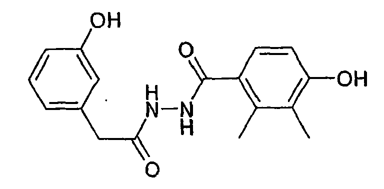 |
| 83 | 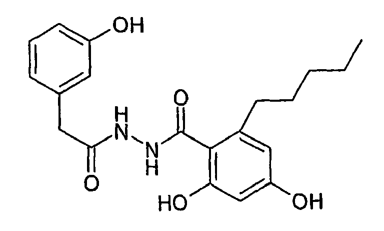 |
| 84 | 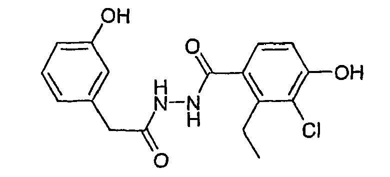 |
| 85 | 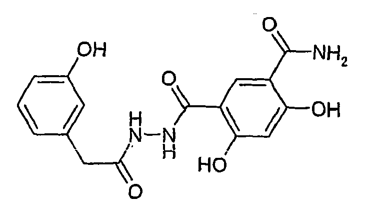 |
| 86 | 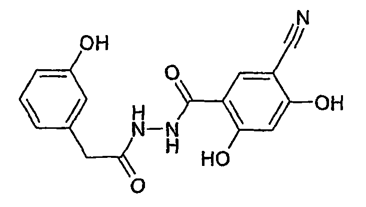 |
| 87 | 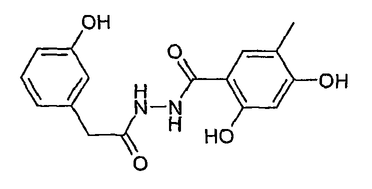 |
| 88 | 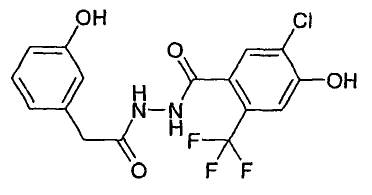 |
sowie ihre pharmazeutisch verwendbaren Derivate, Salze, Solvate und Stereoisomere, einschließlich deren Mischungen in allen Verhältnissen.

18. Verfahren zur Herstellung von Verbindungen der Formel I nach den Ansprüchen 1-17 sowie ihrer pharmazeutisch verwendbaren Derivate, Solvate, Salze und Stereoisomere, **dadurch gekennzeichnet, daß** man
a) eine Verbindung der Formel II 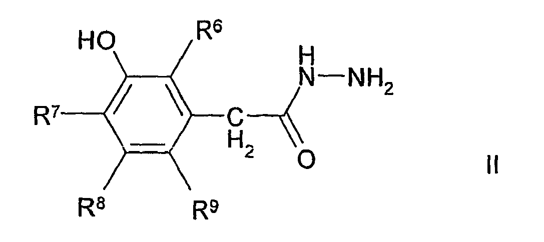 worin
R⁶, R⁷, R⁸ und R⁹ die in Anspruch 1 angegebenen Bedeutungen haben,
mit einer Verbindung der Formel III 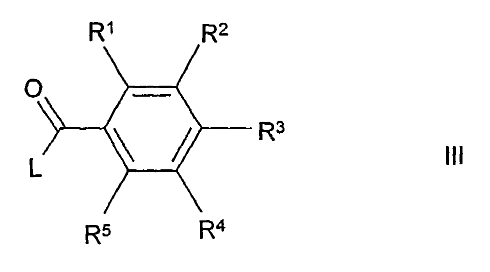 worin
L Cl, Br, I oder eine freie oder reaktionsfähig funktionell abgewandelte OH-Gruppe bedeutet und
R¹, R², R³, R⁴ und R⁵ die in Anspruch 1 angegebenen Bedeutungen haben,
umsetzt,
oder
b) eine Verbindung der Formel IV 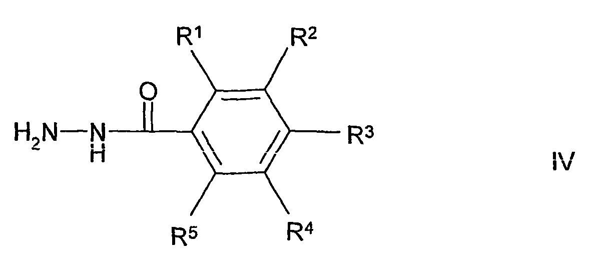 worin
R¹, R², R³, R⁴ und R⁵ die in Anspruch 1 angegebenen Bedeutungen haben,
mit einer Verbindung der Formel V 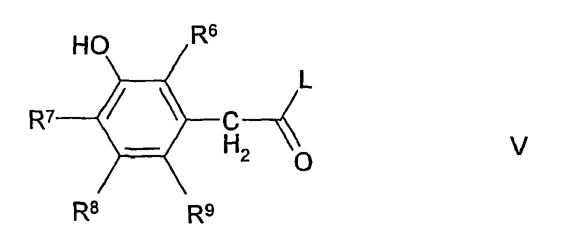 worin
L Cl, Br, I oder eine freie oder reaktionsfähig funktionell abgewandelte OH-Gruppe bedeutet und
R⁶, R⁷, R⁸ und R⁹ die in Anspruch 1 angegebenen Bedeutungen haben,
umsetzt,
oder
c) in einer Verbindung der Formel I einen Rest R¹, R², R³, R⁴, R⁵, R⁶, R⁷, R⁸ und/oder R⁹ in einen anderen Rest R¹, R², R³, R⁴, R⁵, R⁶, R⁷, R⁸ und/oder R⁹ umwandelt,
indem man einen Ether durch Hydrolyse oder Hydrogenolyse spaltet, und/oder eine Base oder Säure der Formel I in eines ihrer Salze umwandelt.

19. Arzneimittel, enthaltend mindestens eine Verbindung nach Anspruch 1-17 und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

20. Verwendung von Verbindungen gemäß Anspruch 1-17, sowie ihrer pharmazeutisch verwendbaren Derivate, Solvate und Stereoisomere, einschließlich deren Mischungen in allen Verhältnissen, zur Herstellung eines Arzneimittels zur Behandlung und/oder Prophylaxe von Krankheiten, bei denen die Hemmung, Regulierung und/oder Modulation der Signaltransduktion von Kinasen eine Rolle spielt.

21. Verwendung nach Anspruch 20, wobei es sich bei der Kinase um SGK handelt.

22. Verwendung nach Anspruch 21 von Verbindungen gemäß Anspruch 1-17, sowie ihrer pharmazeutisch verwendbaren Derivate, Solvate und Stereoisomere, einschließlich deren Mischungen in allen Verhältnissen, zur Herstellung eines Arzneimittels zur Behandlung von Krankheiten, die durch Inhibierung der SGK durch die Verbindungen nach Anspruch 1-17 beeinflußt werden.

23. Verwendung nach Anspruch 22 von Verbindungen gemäß Anspruch 1-17, sowie ihrer pharmazeutisch verwendbaren Derivate, Solvate und Stereoisomere, einschließlich deren Mischungen in allen Verhältnissen, zur Herstellung eines Arzneimittels zur Behandlung oder Vorbeugung von Diabetes, Fettsucht, metabolischem Syndrom (Dyslipidämie), systemischer und pulmonaler Hypertonie, Herzkreislauferkrankungen und Nierenerkrankungen, allgemein bei jeglicher Art von Fibrosen und entzündlichen Prozessen, Krebs, Tumorzellen, Tumormetastasen, Koagulopathien, neuronaler Erregbarkeit, Glaukom, Katarakt, bakteriellen Infektionen sowie in einer antiinfektiösen Therapie, zur Steigerung der Lernfähigkeit und Aufmerksamkeit, sowie zur Behandlung und Prophylaxe von Zellalterung und Stress und zur Behandlung von Tinitus.

24. Verwendung nach Anspruch 23, wobei es sich bei Diabetes um Diabetes mellitus, diabetische Nephropathie, diabetische Neuropathie, diabetische Angiopathie und Mikroangiopathie handelt.

25. Verwendung nach Anspruch 23, wobei es sich bei Herzkreislauferkrankungen um kardiale Fibrosen nach Myokardinfarkt, Herzhypertrophie, Herzinsuffizienz und Arteriosklerose handelt.

26. Verwendung nach Anspruch 23, wobei es sich bei Nierenerkrankungen um Glomerulosklerose, Nephrosklerose, Nephritis, Nephropathie und Störung der Elektrolytausscheidung handelt.

27. Verwendung nach Anspruch 23, wobei es sich bei Fibrosen und entzündlichen Prozessen um Leberzirrhose, Lungenfibrose, fibrosierende Pankreatitis, Rheumatismus und Arthrosen, Morbus Crohn, chronische Bronchitis, Strahlenfibrose, Sklerodermitis, zystische Fibrose, Narbenbildung und Morbus Alzheimer handelt.

28. Arzneimittel enthaltend mindestens eine Verbindung gemäß Anspruch 1-17 und/oder ihre pharmazeutisch verwendbaren Derivate, Solvate und Stereoisomere, einschließlich deren Mischungen in allen Verhältnissen, und mindestens einen weiteren Arzneimittelwirkstoff.

29. Set (Kit), bestehend aus getrennten Packungen von
(a) einer wirksamen Menge an einer Verbindung gemäß Anspruch 1-17 und/oder ihrer pharmazeutisch *verwendbaren* Derivate, Solvate und Stereoisomere, einschließlich deren Mischungen in allen Verhältnissen, und
(b) einer wirksamen Menge eines weiteren Arzneimittelswirkstoffs.

## Claims

1. Compounds of the formula I 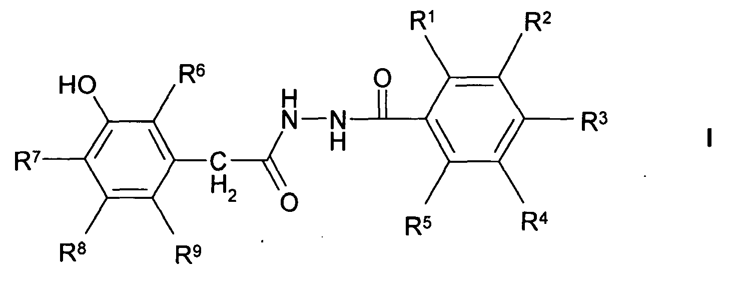 in which
R¹, R², R³, R⁴, R⁵,
R⁶, R⁷, R⁸, R⁹ each, independently of one another, denote H, A, OSO₂A, Hal, NO₂, OR¹⁰, N(R¹⁰)₂, CN, -[C(R¹⁰)₂]ₙCOOR¹⁰, O-[C(R¹⁰)₂]ₒCOOR¹⁰, SO₃H, -[C(R¹⁰)₂]ₙAr, -CO-Ar, O-[C(R¹⁰)₂]ₙAr, -[C(R¹⁰)₂]ₙHet, -[C(R¹⁰)₂]ₙC≡CH, O-[C(R¹⁰)₂]ₙC≡CH, -[C(R¹⁰)₂]ₙCON(R¹⁰)₂, -[C(R¹⁰)₂]ₙCONR¹⁰N(R¹⁰)₂, O-[C(R¹⁰)₂]ₙCON(R¹⁰)₂, O-[C(R¹⁰)₂]ₒCONR¹⁰N(R¹⁰)₂, NR¹⁰COA, NR¹⁰CON(R¹⁰)₂, NR¹⁰SO₂A, N(SO₂A)₂, COR¹⁰, S(O)ₘAr, SO₂NR¹⁰ or S(O)ₘA,
R¹ and R², R² and R³,
R³ and R⁴ or R⁴ and R⁵ together also denote CH=CH-CH=CH,
A denotes unbranched or branched alkyl having 1-6 C atoms, in which 1-7 H atoms may be replaced by F, or cyclic alkyl having 3-7 C atoms,
Ar denotes phenyl, naphthyl or biphenyl, each of which is unsubstituted or mono-, di- or trisubstituted by Hal, A, OR¹⁰, N(R¹⁰)₂, NO₂, CN, phenyl, CON(R¹⁰)₂, NR¹⁰COA, NR¹⁰CON(R¹⁰)₂, NR¹⁰SO₂A, COR¹⁰, SO₂N(R¹⁰)₂, S(O)ₘA, -[C(R¹⁰)₂]ₙ-COOR¹⁰ and/or -O[C(R¹⁰)₂]ₒ-COOR¹⁰,
Het denotes a mono- or bicyclic saturated, unsaturated or aromatic heterocycle having 1 to 4 N, O and/or S atoms, which may be mono-, di- or trisubstituted by Hal, A, OR¹⁰, N(R¹⁰)₂, NO₂, CN, COOR¹⁰, CON(R¹⁰)₂, NR¹⁰COA, NR¹⁰SO₂A, COR¹⁰, SO₂NR¹⁰, S(O)ₘA, =S, =NR¹⁰ and/or =O (carbonyl oxygen),
R¹⁰ denotes H or A,
Hal denotes F, Cl, Br or I,
m denotes 0, 1 or 2,
n denotes 0, 1, 2 or 3,
o denotes 1, 2 or 3,
and pharmaceutically usable derivatives, salts, solvates and stereoisomers thereof, including mixtures thereof in all ratios.

2. Compounds according to Claim 1 in which
R¹ denotes H, A, Hal, NO₂, OR¹⁰, -[C(R¹⁰)₂]ₙAr or O-[C(R¹⁰)₂]ₙAr, and pharmaceutically usable derivatives, salts, solvates and stereoisomers thereof, including mixtures thereof in all ratios.

3. Compounds according to Claim 1 or 2 in which
R² denotes H, A, Hal, CN, NO₂, OR¹⁰ -[C(R¹⁰)₂]ₙAr or O-[C(R¹⁰)₂]ₙAr,
and pharmaceutically usable derivatives, salts, solvates and stereoisomers thereof, including mixtures thereof in all ratios.

4. Compounds according to one or more of Claims 1-3 in which
R³ denotes H, A, Hal, NO₂, OR¹⁰, -[C(R¹⁰)₂]ₙAr, O-[C(R¹⁰)₂]ₙAr, -[C(R¹⁰)₂]ₙCOOR¹⁰ or S(O)ₘA,
and pharmaceutically usable derivatives, salts, solvates and stereoisomers thereof, including mixtures thereof in all ratios.

5. Compounds according to one or more of Claims 1-4 in which
R⁴ denotes H, A, Hal, CONH₂, CN, NO₂ or OR¹⁰,
and pharmaceutically usable derivatives, salts, solvates and stereoisomers thereof, including mixtures thereof in all ratios.

6. Compounds according to one or more of Claims 1-5 in which
R⁵ denotes H, A, Hal, OR¹⁰, -[C(R¹⁰)₂]ₙAr or O-[C(R¹⁰)₂]ₙAr, and pharmaceutically usable derivatives, salts, solvates and stereoisomers thereof, including mixtures thereof in all ratios.

7. Compounds according to one or more of Claims 1-6 in which
R⁶ denotes H or A,
and pharmaceutically usable derivatives, salts, solvates and stereoisomers thereof, including mixtures thereof in all ratios.

8. Compounds according to one or more of Claims 1-7 in which
R⁷ denotes H, A or OR¹⁰,
and pharmaceutically usable derivatives, salts, solvates and stereoisomers thereof, including mixtures thereof in all ratios.

9. Compounds according to one or more of Claims 1-8 in which
R⁸ denotes H, A or OR¹⁰,
and pharmaceutically usable derivatives, salts, solvates and stereoisomers thereof, including mixtures thereof in all ratios.

10. Compounds according to one or more of Claims 1-9 in which
R⁹ denotes H or A,
and pharmaceutically usable derivatives, salts, solvates and stereoisomers thereof, including mixtures thereof in all ratios.

11. Compounds according to one or more of Claims 1-10 in which
Ar denotes phenyl which is unsubstituted or mono-, di- or trisubstituted by Hal and/or A,
and pharmaceutically usable derivatives, salts, solvates and stereoisomers thereof, including mixtures thereof in all ratios.

12. Compounds according to one or more of Claims 1-11 in which
Het denotes a monocyclic saturated, unsaturated or aromatic heterocycle having 1 to 2 N and/or O atoms, which may be unsubstituted or mono-, di- or trisubstituted by A, Hal, OH and/or OA,
and pharmaceutically usable derivatives, salts, solvates and stereoisomers thereof, including mixtures thereof in all ratios.

13. Compounds according to one or more of Claims 1-12 in which
Het denotes a monocyclic saturated heterocycle having 1 to 2 N and/or O atoms, which may be unsubstituted or mono- or disubstituted by A,
and pharmaceutically usable derivatives, salts, solvates and stereoisomers thereof, including mixtures thereof in all ratios.

14. Compounds according to one or more of Claims 1-13 in which
Het denotes furyl, thienyl, pyrrolyl, imidazolyl, pyridyl, pyrimidinyl, pyrazolyl, thiazolyl, indolyl, pyrrolidinyl, piperidinyl, morpholinyl or piperazinyl, each of which is unsubstituted or mono-, di- or trisubstituted by A, Hal, OH and/or OA,
and pharmaceutically usable derivatives, salts, solvates and stereoisomers thereof, including mixtures thereof in all ratios.

15. Compounds according to one or more of Claims 1-14 in which
R¹ denotes H, A, Hal, NO₂, OR¹⁰, -[C(R¹⁰)₂]ₙAr or O-[C(R¹⁰)₂]ₙAr,
R² denotes H, A, Hal, CN, N(R¹⁰)₂, NO₂, OR¹⁰, -[C(R¹⁰)₂]ₙAr or O-[C(R¹⁰)₂]ₙAr,
R³ denotes H, A, Hal, NO₂, OR¹⁰, -[C(R¹⁰)₂]ₙAr, O-[C(R¹⁰)₂]ₙAr, -[C(R¹⁰)₂]ₙCOOR¹⁰ or S(O)ₘA,
R⁴ denotes H, A, Hal, CONH₂, CN, NO₂ or OR¹⁰,
R⁵ denotes H, A, Hal, OR¹⁰, -[C(R¹⁰)₂]ₙAr or O-[C(R¹⁰)₂]ₙAr,
R⁶ denotes H,
R⁷ denotes H or OR¹⁰,
R⁸ denotes H or OR¹⁰,
R⁹ denotes H,
R¹ and R², R² and R³,
R³ and R⁴ or R⁴ and R⁵ together also denote CH=CH-CH=CH,
A denotes unbranched or branched alkyl having 1-6 C atoms, in which 1-7 H atoms may be replaced by F,
Ar denotes phenyl which is unsubstituted or mono-, di- or trisubstituted by Hal and/or A,
R¹⁰ denotes H or A,
Hal denotes F, Cl, Br or I,
m denotes 0, 1 or 2,
n denotes 0, 1, 2 or 3,
and pharmaceutically usable derivatives, salts, solvates and stereoisomers thereof, including mixtures thereof in all ratios.

16. Compounds according to one or more of Claims 1-15 in which
R¹ denotes OH, A or Hal,
R² denotes H, A or Hal,
R³ denotes OH,
R⁴ denotes H, A or Hal,
R⁵ denotes H or OH,
R⁶ denotes H,
R⁷ denotes H,
R⁸ denotes H,
R⁹ denotes H,
R¹ and R², R² and R³,
R³ and R⁴ or R⁴ and R⁵ together also denote CH=CH-CH=CH,
A denotes unbranched or branched alkyl having 1-6 C atoms, in which 1-7 H atoms may be replaced by F,
Hal denotes F, Cl, Br or I,
and pharmaceutically usable derivatives, salts, solvates and stereoisomers thereof, including mixtures thereof in all ratios.

17. Compounds according to Claim 1 selected from the group
| No. | Structural formula |
|---|---|
| 1 | 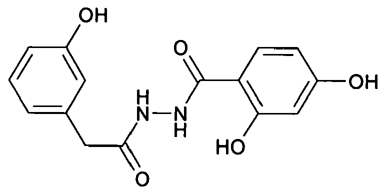 |
| 2 | 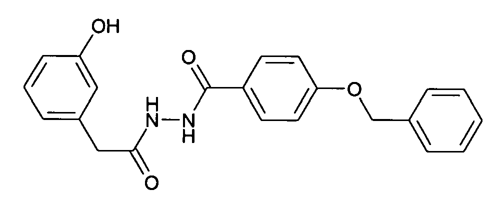 |
| 3 | 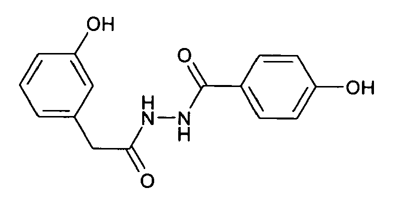 |
| 4 | 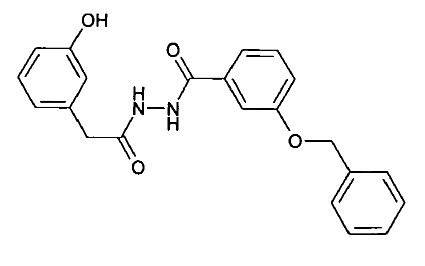 |
| 5 | 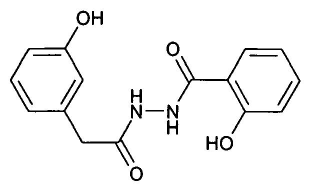 |
| 6 | 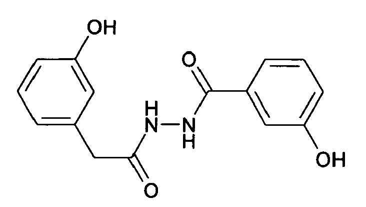 |
| 7 | 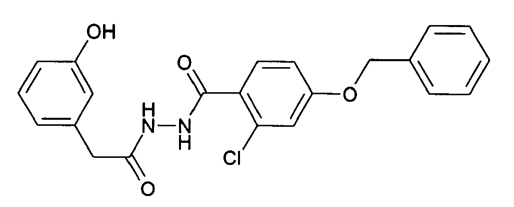 |
| 8 | 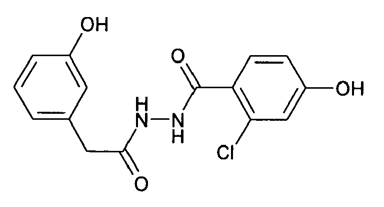 |
| 9 | 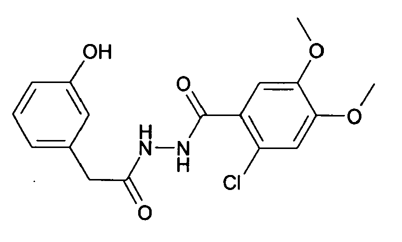 |
| 10 | 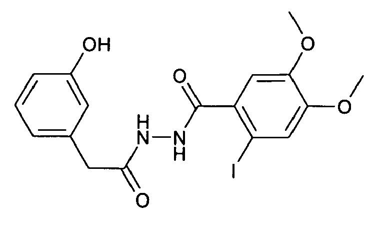 |
| 11 | 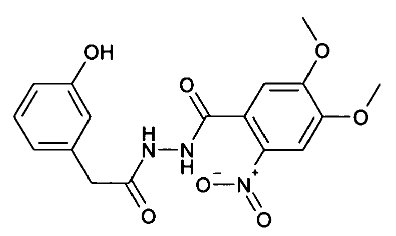 |
| 12 | 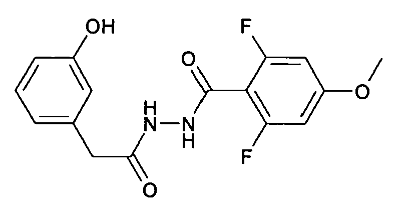 |
| 13 | 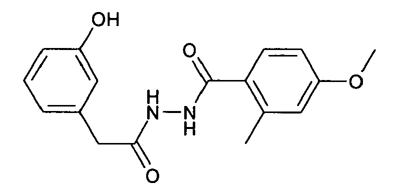 |
| 14 | 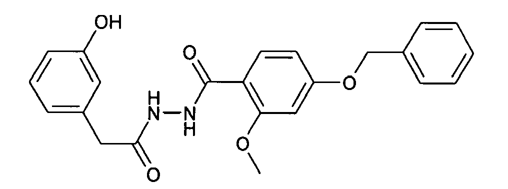 |
| 15 | 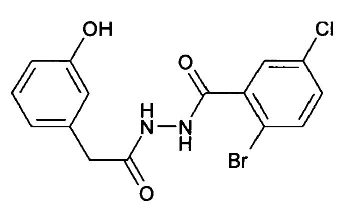 |
| 16 | 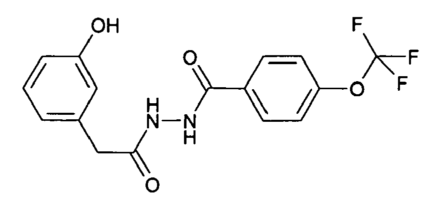 |
| 17 | 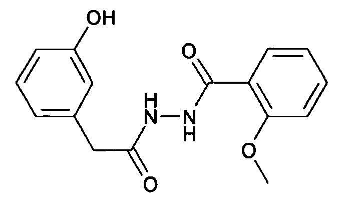 |
| 18 | 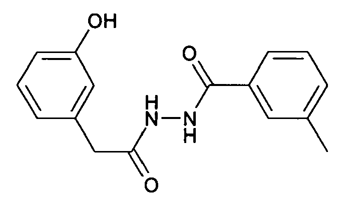 |
| 19 | 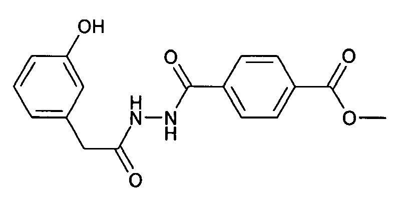 |
| 20 | 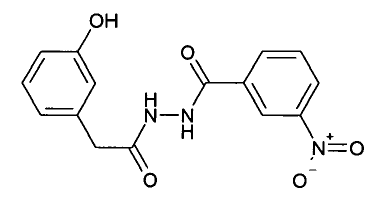 |
| 21 | 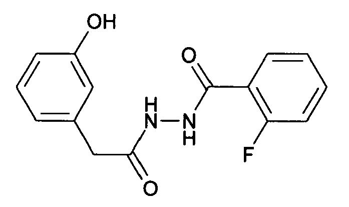 |
| 22 | 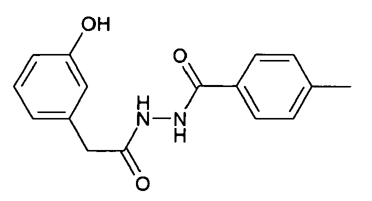 |
| 23 | 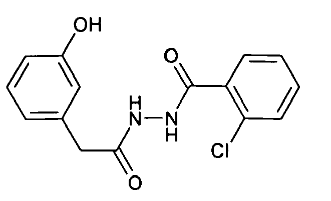 |
| 24 | 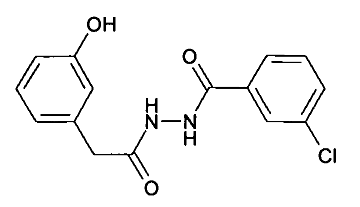 |
| 25 | 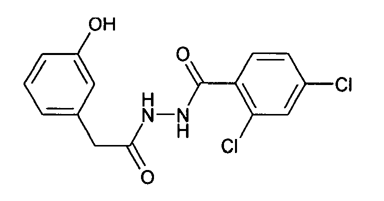 |
| 26 | 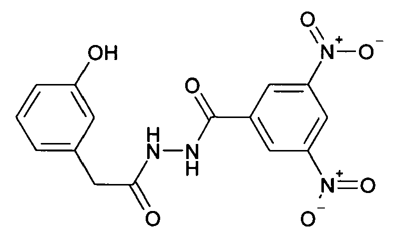 |
| 27 | 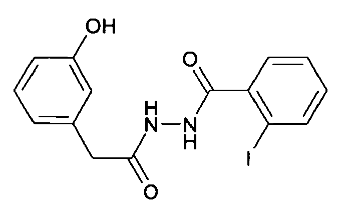 |
| 28 | 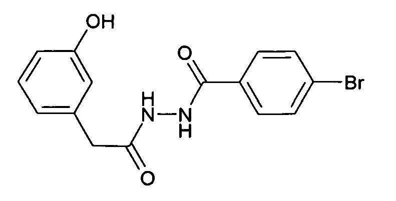 |
| 29 | 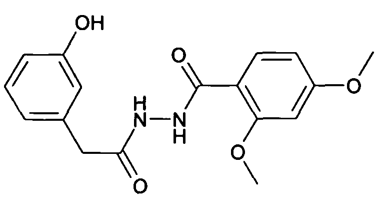 |
| 30 | 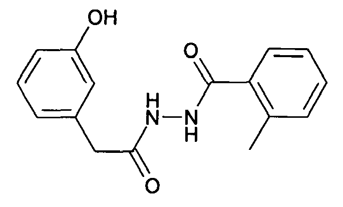 |
| 31 | 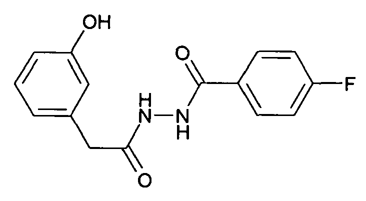 |
| 32 | 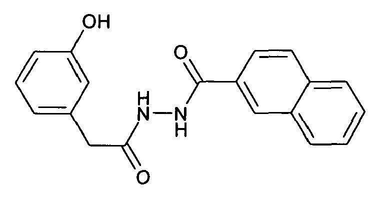 |
| 33 | 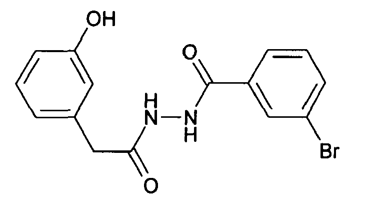 |
| 34 | 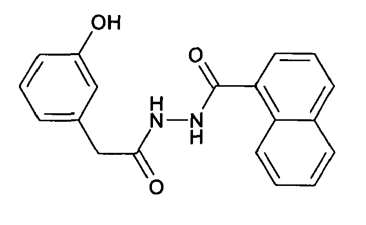 |
| 35 | 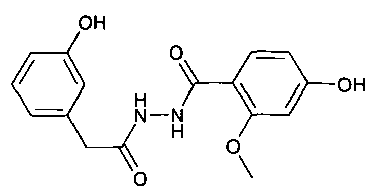 |
| 36 | 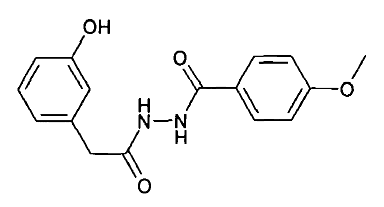 |
| 37 | 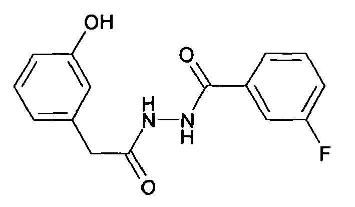 |
| 38 | 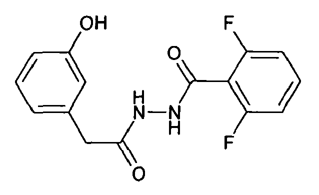 |
| 39 | 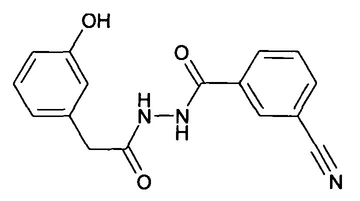 |
| 40 | 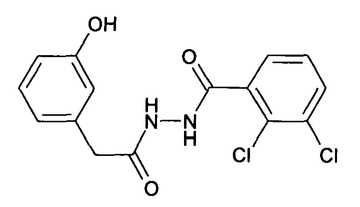 |
| 41 | 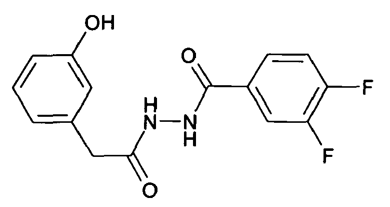 |
| 42 | 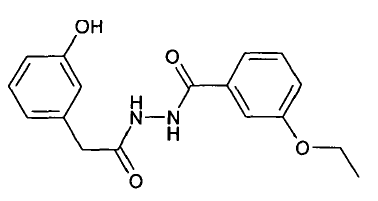 |
| 43 | 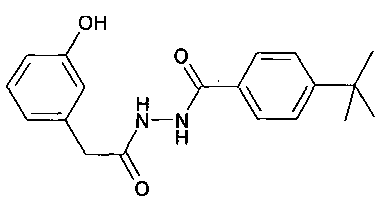 |
| 44 | 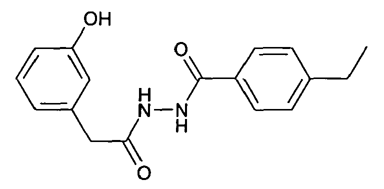 |
| 45 | 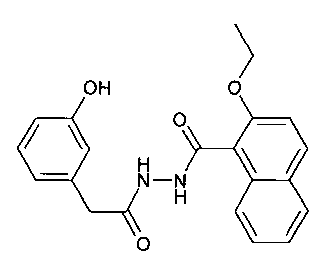 |
| 46 | 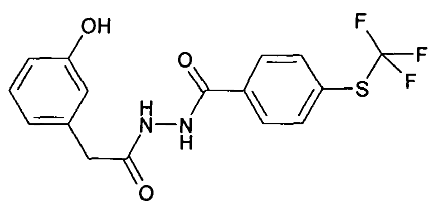 |
| 47 | 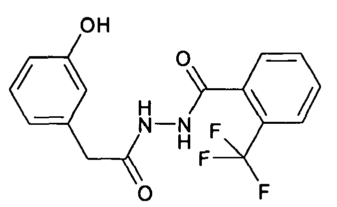 |
| 48 | 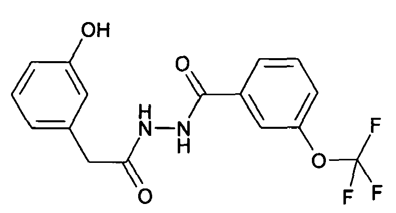 |
| 49 | 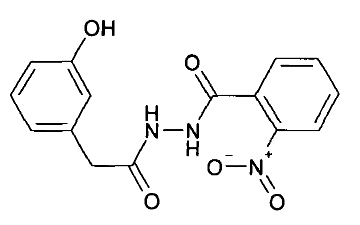 |
| 50 | 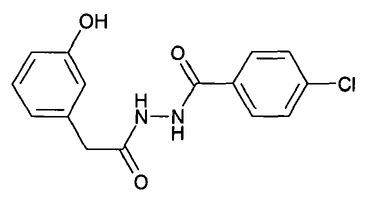 |
| 51 | 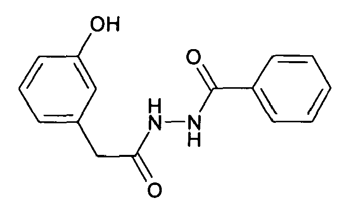 |
| 52 | 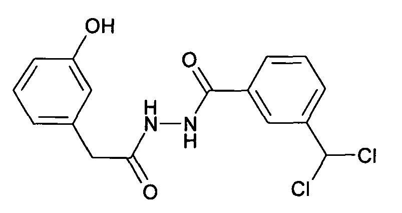 |
| 53 | 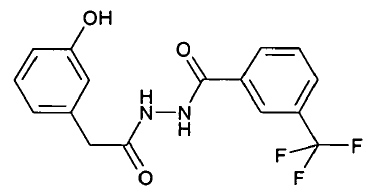 |
| 54 | 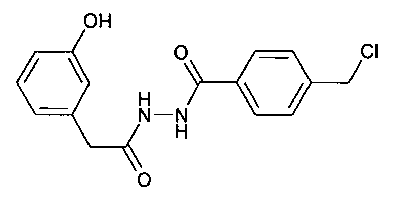 |
| 55 | 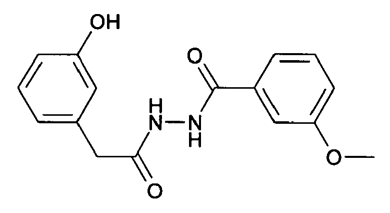 |
| 56 | 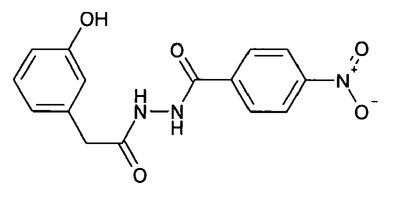 |
| 57 | 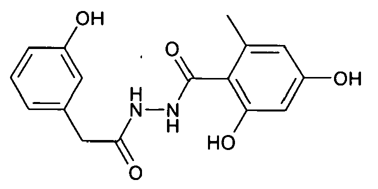 |
| 58 | 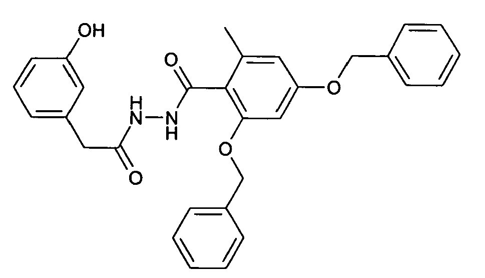 |
| 59 | 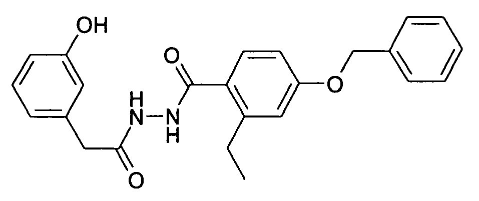 |
| 60 | 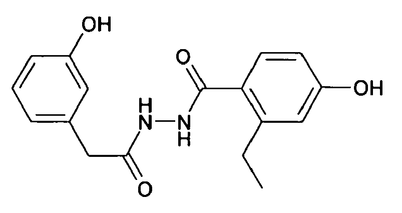 |
| 61 | 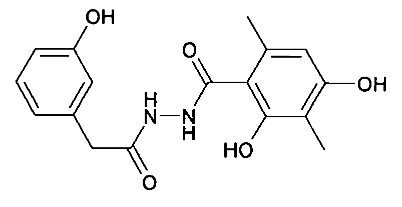 |
| 62 | 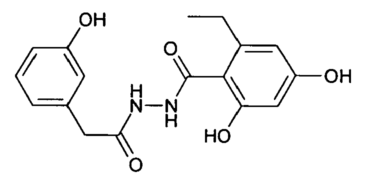 |
| 63 | 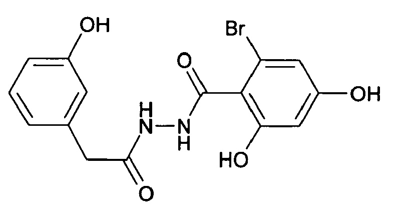 |
| 64 | 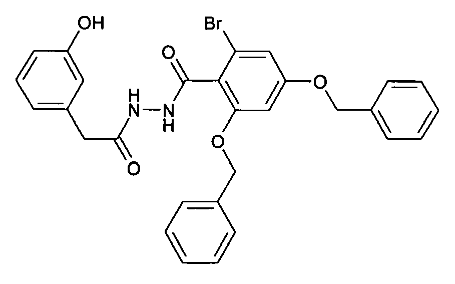 |
| 65 | 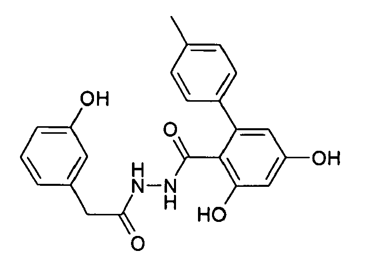 |
| 66 | 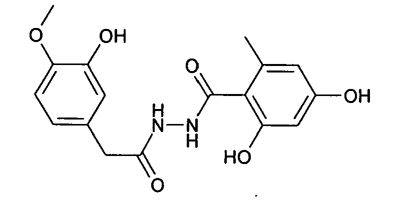 |
| 67 | 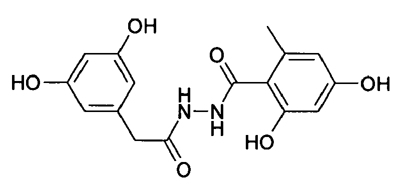 |
| 68 | 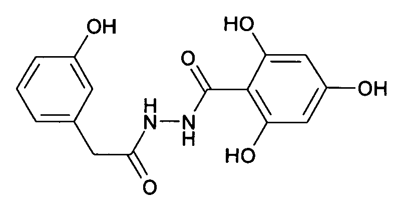 |
| 69 | 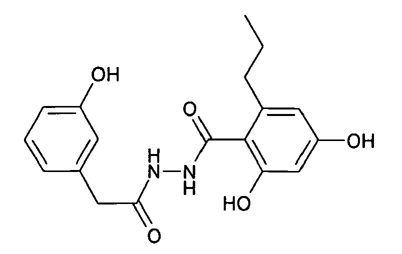 |
| 70 | 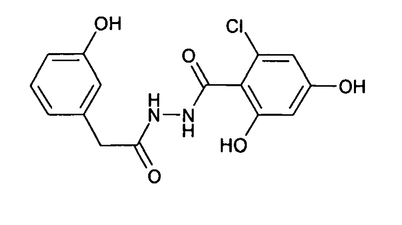 |
| 71 | 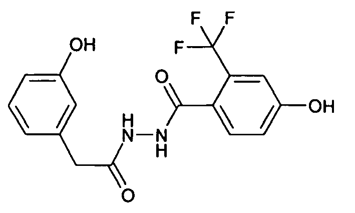 |
| 72 | 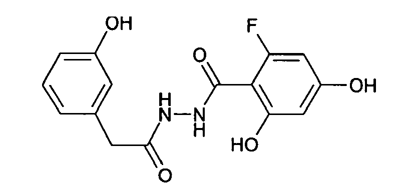 |
| 73 | 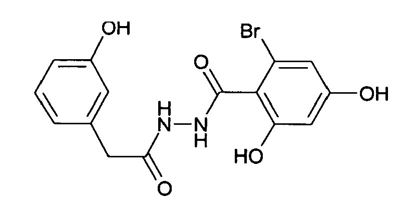 |
| 74 | 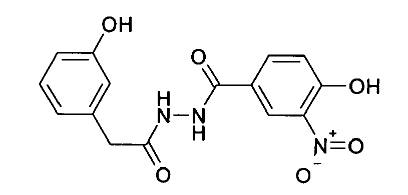 |
| 75 | 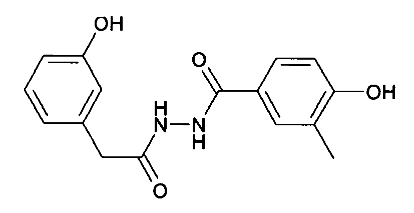 |
| 76 | 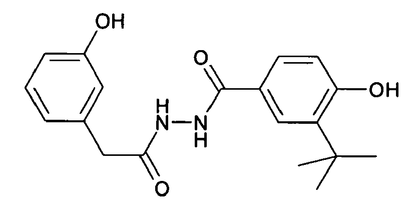 |
| 77 | 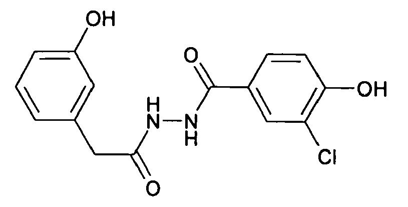 |
| 78 | 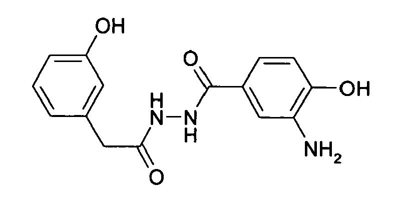 |
| 79 | 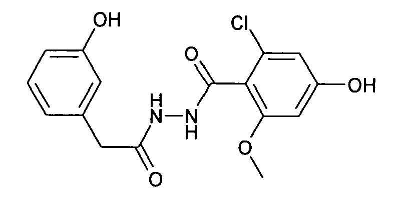 |
| 80 | 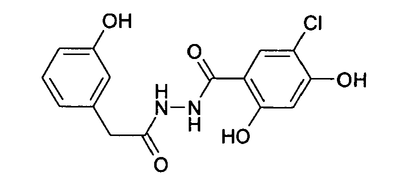 |
| 81 | 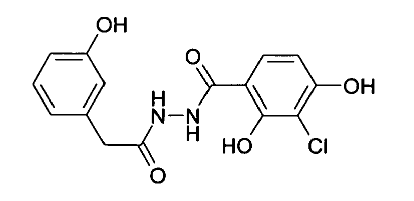 |
| 82 | 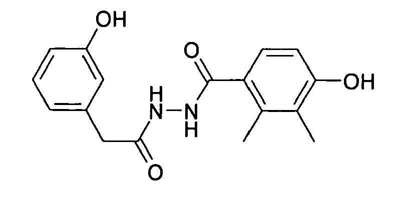 |
| 83 | 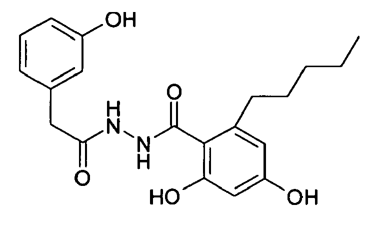 |
| 84 | 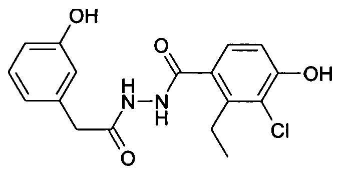 |
| 85 | 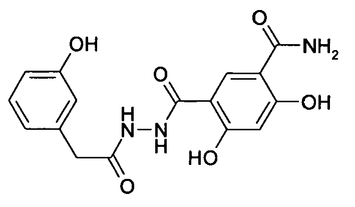 |
| 86 | 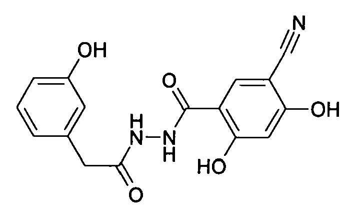 |
| 87 | 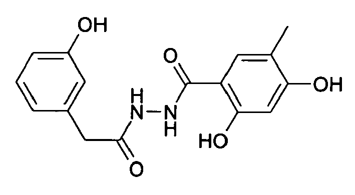 |
| 88 | 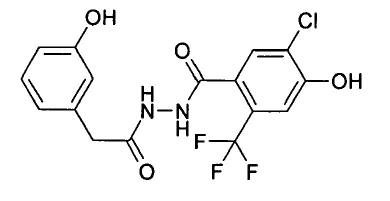 |
and pharmaceutically usable derivatives, salts, solvates and stereoisomers thereof, including mixtures thereof in all ratios.

18. Process for the preparation of compounds of the formula I according to Claims 1-17 and pharmaceutically usable derivatives, solvates, salts and stereoisomers thereof, **characterised in that**
a) a compound of the formula II 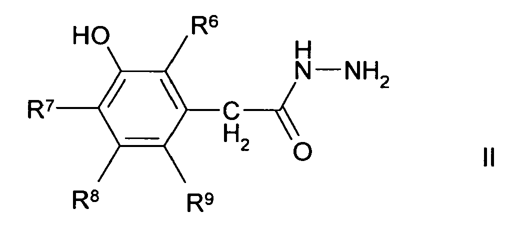 in which
R⁶, R⁷, R⁸ and R⁹ have the meanings indicated in Claim 1,
is reacted with a compound of the formula III 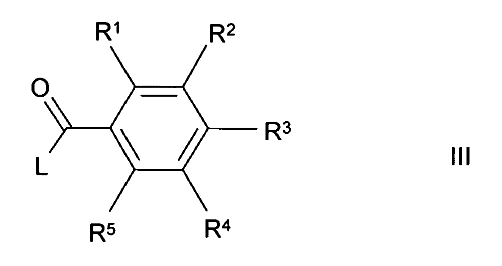 in which
L denotes Cl, Br, I or a free or reactively functionally modified OH group and
R¹, R², R³, R⁴ and R⁵ have the meanings indicated in Claim 1,
or
b) a compound of the formula IV 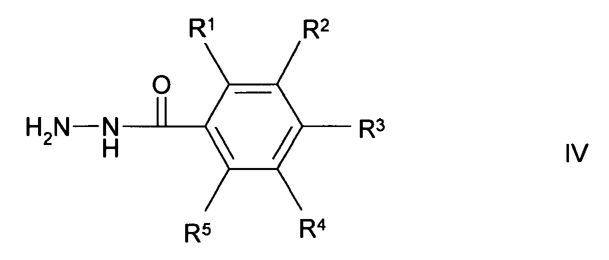 in which
R¹, R², R³, R⁴ and R⁵ have the meanings indicated in Claim 1, is reacted with a compound of the formula V 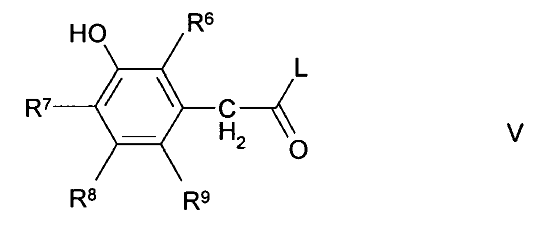 in which
L denotes Cl, Br, I or a free or reactively functionally modified OH group and
R⁶, R⁷, R⁸ and R⁹ have the meanings indicated in Claim 1,
or
c) a radical R¹, R², R³, R⁴, R⁵, R⁶, R⁷, R⁸ and/or R⁹ in a compound of the formula I is converted into another radical R¹, R², R³, R⁴, R⁵, R⁶, R⁷, R⁸ and/or R⁹
by cleaving an ether by hydrolysis or hydrogenolysis,
and/or a base or acid of the formula I is converted into one of its salts.

19. Medicaments comprising at least one compound according to Claim 1-17 and/or pharmaceutically usable derivatives, solvates and stereoisomers thereof, including mixtures thereof in all ratios, and optionally excipients and/or adjuvants.

20. Use of compounds according to Claim 1-17, and pharmaceutically usable derivatives, solvates and stereoisomers thereof, including mixtures thereof in all ratios, for the preparation of a medicament for the treatment and/or prophylaxis of diseases in which the inhibition, regulation and/or modulation of kinase signal transduction plays a role.

21. Use according to Claim 20, where the kinase is SGK.

22. Use according to Claim 21 of compounds according to Claim 1-17, and pharmaceutically usable derivatives, solvates and stereoisomers thereof, including mixtures thereof in all ratios, for the preparation of a medicament for the treatment of diseases which are influenced by inhibition of SGKs by the compounds according to Claim 1-17.

23. Use according to Claim 22 of compounds according to Claim 1-17, and pharmaceutically usable derivatives, solvates and stereoisomers thereof, including mixtures thereof in all ratios, for the preparation of a medicament for the treatment or prevention of diabetes, obesity, metabolic syndrome (dyslipidaemia), systemic and pulmonary hypertension, cardiovascular diseases and renal diseases, generally in all types of fibroses and inflammatory processes, cancer, tumour cells, tumour metastases, coagulopathies, neuronal excitability, glaucoma, cataract, bacterial infections and in antiinfection therapy, for increasing learning ability and attention, and for the treatment and prophylaxis of cell ageing and stress and for the treatment of tinnitus.

24. Use according to Claim 23, where diabetes is diabetes mellitus, diabetic nephropathy, diabetic neuropathy, diabetic angiopathy and microangiopathy.

25. Use according to Claim 23, where cardiovascular diseases are cardiac fibroses after myocardial infarction, cardiac hypertrophy, cardiac insufficiency and arteriosclerosis.

26. Use according to Claim 23, where renal diseases are glomerulosclerosis, nephrosclerosis, nephritis, nephropathy and impaired electrolyte excretion.

27. Use according to Claim 23, where fibroses and inflammatory processes are liver cirrhosis, pulmonary fibrosis, fibrosing pancreatitis, rheumatism and arthroses, Crohn's disease, chronic bronchitis, radiation fibrosis, sclerormatitis, cystic fibrosis, scarring and Alzheimer's disease.

28. Medicaments comprising at least one compound according to Claim 1-17 and/or pharmaceutically usable derivatives, solvates and stereoisomers thereof, including mixtures thereof in all ratios, and at least one further medicament active compound.

29. Set (kit) consisting of separate packs of
(a) an effective amount of a compound according to Claim 1-17 and/or pharmaceutically usable derivatives, solvates and stereoisomers thereof, including mixtures thereof in all ratios, and
(b) an effective amount of a further medicament active compound.

## Revendications

1. Composés de formule I 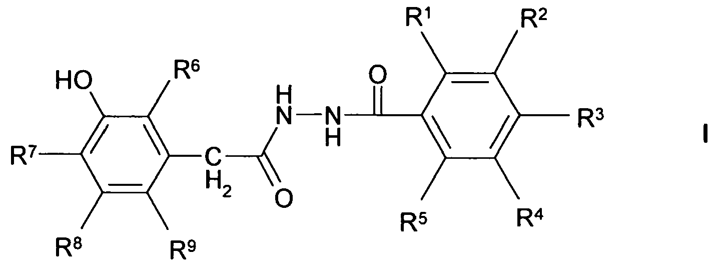 dans laquelle
R¹, R², R³, R⁴, R⁵,
R⁶, R⁷, R⁸, R⁹ désignent chacun, indépendamment les uns des autres, **H,** A, OSO₂A, Hal, NO₂, OR¹⁰, N(R¹⁰)₂, **CN,** -[C(R¹⁰)₂]ₙCOOR¹⁰, O-[C(R¹⁰)₂]ₒCOOR¹⁰, SO₃H, -[C(R¹⁰)₂]ₙAr, -CO-Ar, O-[C(R¹⁰)₂]ₙAr, -[C(R¹⁰)₂]ₙHét, -[C(R¹⁰)₂]ₙC≡CH, O-[C(R¹⁰)₂]ₙC≡CH, -[C(R¹⁰)₂]ₙCON(R¹⁰)₂, -[C(R¹⁰)₂]ₙCONR¹⁰N(R¹⁰)₂, O-[C(R¹⁰)₂]ₙCON(R¹⁰)₂, O-[C(R¹⁰)₂]ₒCONR¹⁰N(R¹⁰)₂. NR¹⁰COA, NR¹⁰CON(R¹⁰)₂, NR¹⁰SO₂A, N(SO₂A)₂, COR'°, S(O)ₘAr, SO₂NR¹⁰ ou S(O)ₘA,
R¹ et R² R² et R³,
R³ et R⁴ ou R⁴ et R⁵ ensemble désignent aussi CH=CH-CH=CH,
A désigne alkyle linéaire ou ramifié ayant 1-6 atomes de C, dans lequel 1-7 atomes de H peuvent être remplacés par F, ou alkyle cyclique ayant 3-7 atomes de C,
Ar désigne phényle, naphtyle ou biphényle, chacun desquels étant non substitué ou mono-, di- ou trisubstitué par Hal, A, OR¹⁰, N(R¹⁰)₂, NO₂, CN, phényle, CON(R¹⁰)₂, NR¹⁰COA, NR¹⁰CON(R¹⁰)₂, NR¹⁰SO₂A, COR¹⁰, SO₂N(R¹⁰)₂, S(O)ₘA, -[C(R¹⁰)₂]ₙ-COOR¹⁰ et/ou -O[C(R¹⁰)₂]ₒ-COOR¹⁰,
Hét désigne un hétérocycle mono- ou bicyclique, saturé, insaturé ou aromatique ayant de 1 à 4 atomes de N, O et/ou S, qui peut être mono-, di ou trisubstitué par Hal, A, OR¹⁰, N(R¹⁰)₂, NO₂, CN, COOR¹⁰, CON(R¹⁰)₂, NR¹⁰COA, NR¹⁰SO₂A, COR¹⁰, SO₂NR¹⁰, S(O)ₘA, =S, =NR¹⁰ et/ou =O (oxygène carbonyle),
R¹⁰ désigne H ou A,
Hal désigne F, Cl, Br ou I,
m désigne 0, 1 ou 2,
n désigne 0, 1, 2 ou 3,
o désigne 1, 2 ou 3,
ainsi que les dérivés, sels, solvats et stéréoisomères pharmaceutiquement utilisables de ceux-ci, y compris des mélanges de ceux-ci en toutes proportions.

2. Composés selon la revendication 1, dans lesquels
R¹ désigne H, A, Hal, NO₂, OR¹⁰, -[C(R¹⁰)₂]ₙAr ou O-[C(R¹⁰)₂]ₙAr, ainsi que les dérivés, sels, solvats et stéréoisomères pharmaceutiquement utilisables de ceux-ci, y compris des mélanges de ceux-ci en toutes proportions.

3. Composés selon la revendication 1 ou 2, dans lesquels
R² désigne H, A, Hal, CN, NO₂, OR¹⁰, -[C(R¹⁰)₂]ₙAr ou O-[C(R¹⁰)₂]ₙAr,
ainsi que les dérivés, sels, solvats et stéréoisomères pharmaceutiquement utilisables de ceux-ci, y compris des mélanges de ceux-ci en toutes proportions.

4. Composés selon l'une ou plusieurs parmi les revendications 1-3,
dans lesquels
R³ désigne H, A, Hal, NO₂, OR¹⁰, -[C(R¹⁰)₂]ₙAr, O-[C(R¹⁰)₂]ₙAr, -[C(R¹⁰)₂]ₙCOOR¹⁰ ou S(O)ₘA,
ainsi que les dérivés, sels, solvats et stéréoisomères pharmaceutiquement utilisables de ceux-ci, y compris des mélanges de ceux-ci en toutes proportions.

5. Composés selon l'une ou plusieurs parmi les revendications 1-4,
dans lesquels
R⁴ désigne H, A, Hal, CONH₂, CN, NO₂ ou OR¹⁰,
ainsi que les dérivés, sels, solvats et stéréoisomères pharmaceutiquement utilisables de ceux-ci, y compris des mélanges de ceux-ci en toutes proportions.

6. Composés selon l'une ou plusieurs parmi les revendications 1-5,
dans lesquels
R⁵ désigne H, A, Hal, OR¹⁰, -[C(R¹⁰)₂]ₙAr ou O-[C(R¹⁰)₂]ₙAr, ainsi que les dérivés, sels, solvats et stéréoisomères pharmaceutiquement utilisables de ceux-ci, y compris des mélanges de ceux-ci en toutes proportions.

7. Composés selon l'une ou plusieurs parmi les revendications 1-6,
dans lesquels
R⁶ désigne H ou A,
ainsi que les dérivés, sels, solvats et stéréoisomères pharmaceutiquement utilisables de ceux-ci, y compris des mélanges de ceux-ci en toutes proportions.

8. Composés selon l'une ou plusieurs parmi les revendications 1-7,
dans lesquels
R⁷ désigne H, A ou OR¹⁰,
ainsi que les dérivés, sels, solvats et stéréoisomères pharmaceutiquement utilisables de ceux-ci, y compris des mélanges de ceux-ci en toutes proportions.

9. Composés selon l'une ou plusieurs parmi les revendications 1-8,
dans lesquels
R⁸ désigne H, A ou OR¹⁰,
ainsi que les dérivés, sels, solvats et stéréoisomères pharmaceutiquement utilisables de ceux-ci, y compris des mélanges de ceux-ci en toutes proportions.

10. Composés selon l'une ou plusieurs parmi les revendications 1-9,
dans lesquels
R⁹ désigne H ou A,
ainsi que les dérivés, sels, solvats et stéréoisomères pharmaceutiquement utilisables de ceux-ci, y compris des mélanges de ceux-ci en toutes proportions.

11. Composés selon l'une ou plusieurs parmi les revendications 1-10,
dans lesquels
Ar désigne phényle qui est non substitué ou mono-, di- ou trisubstitué par Hal et/ou A,
ainsi que les dérivés, sels, solvats et stéréoisomères pharmaceutiquement utilisables de ceux-ci, y compris des mélanges de ceux-ci en toutes proportions.

12. Composés selon l'une ou plusieurs parmi les revendications 1-11,
dans lesquels
Hét désigne un hétérocycle monocyclique saturé, insaturé ou aromatique ayant de 1 à 2 atomes de N et/ou O, qui peut être non substitué ou mono-, di- ou trisubstitué par A, Hal, OH et/ou OA,
ainsi que les dérivés, sels, solvats et stéréoisomères pharmaceutiquement utilisables de ceux-ci, y compris des mélanges de ceux-ci en toutes proportions.

13. Composés selon l'une ou plusieurs parmi les revendications 1-12,
dans lesquels
Hét désigne un hétérocycle monocyclique saturé ayant de 1 à 2 atomes de N et/ou O, qui peut être non substitué ou mono- ou disubstitué par A,
ainsi que les dérivés, sels, solvats et stéréoisomères pharmaceutiquement utilisables de ceux-ci, y compris des mélanges de ceux-ci en toutes proportions.

14. Composés selon l'une ou plusieurs parmi les revendications 1-13,
dans lesquels
Hét désigne furyle, thiényle, pyrrolyle, imidazolyle, pyridinyle, pyrimidinyle, pyrazolyle, thiazolyle, indolyle, pyrrolidinyle, pipéridinyle, morpholinyle ou pipérazinyle, chacun desquels étant non substitué ou mono-, di- ou trisubstitué par A, Hal, OH et/ou OA,
ainsi que les dérivés, sels, solvats et stéréoisomères pharmaceutiquement utilisables de ceux-ci, y compris des mélanges de ceux-ci en toutes proportions.

15. Composés selon l'une ou plusieurs parmi les revendications 1-14,
dans lesquels
R¹ désigne H, A, Hal, NO₂, OR¹⁰, -[C(R¹⁰)₂]ₙAr ou O-[C(R¹⁰)₂]ₙAr,
R² désigne H, A, Hal, CN, N(R¹⁰)₂, NO₂, OR¹⁰, -[C(R¹⁰)₂]ₙAr ou O-[C(R¹⁰)₂]ₙAr,
R³ désigne H, A, Hal, NO₂, OR¹⁰, -[C(R¹⁰)₂]ₙAr, O-[C(R¹⁰)₂]ₙAr, -[C(R¹⁰)₂]ₙCOOR¹⁰ ou S(O)ₘA,
R⁴ désigne H, A, Hal, CONH₂, CN, NO₂ ou OR¹⁰,
R⁵ désigne H, A, Hal, OR¹⁰, -[C(R¹⁰)₂]ₙAr ou O-[C(R¹⁰)₂]ₙAr,
R⁶ désigne H,
R⁷ désigne H ou OR¹⁰,
R⁸ désigne H ou OR¹⁰,
R⁹ désigne H,
R¹ et R² R² et R³,
R³ et R⁴ ou R⁴ et R⁵ ensemble désignent aussi CH=CH-CH=CH,
A désigne alkyle linéaire ou ramifié ayant 1-6 atomes de C, dans lequel 1-7 atomes de H peuvent être remplacés par F,
Ar désigne phényle qui est non substitué ou mono-, di- ou trisubstitué par Hal et/ou A,
R¹⁰ désigne H ou A,
Hal désigne F, Cl, Br ou I,
m désigne 0, 1 ou 2,
n désigne 0, 1, 2 ou 3,
ainsi que les dérivés, sels, solvats et stéréoisomères pharmaceutiquement utilisables de ceux-ci, y compris des mélanges de ceux-ci en toutes proportions.

16. Composés selon l'une ou plusieurs parmi les revendications 1-15,
dans lesquels
R¹ désigne OH, A ou Hal,
R² désigne H, A ou Hal,
R³ désigne OH,
R⁴ désigne H, A ou Hal,
R⁵ désigne H ou OH,
R⁶ désigne H,
R⁷ désigne H,
R⁸ désigne H,
R⁹ désigne H,
R¹ et R², R² et R³,
R³ et R⁴ ou R⁴ et R⁵ ensemble désignent aussi CH=CH-CH=CH,
A désigne alkyle linéaire ou ramifié ayant 1-6 atomes de C, dans lequel 1-7 atomes de H peuvent être remplacés par F,
Hal désigne F, Cl, Br ou I,
ainsi que les dérivés, sels, solvats et stéréoisomères pharmaceutiquement utilisables de ceux-ci, y compris des mélanges de ceux-ci en toutes proportions.

17. Composés selon la revendication 1, sélectionnés parmi le groupe
| N° | Formule structurale |
|---|---|
| 1 | 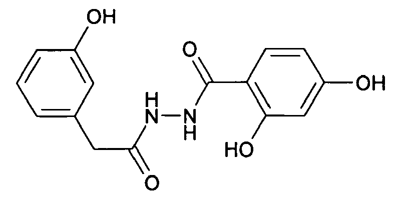 |
| 2 | 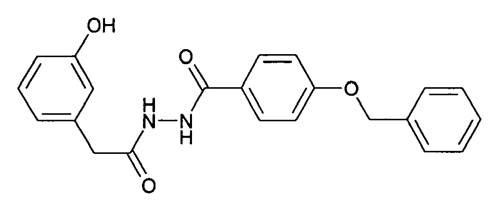 |
| 3 | 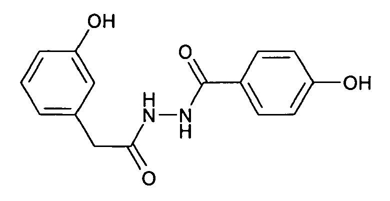 |
| 4 | 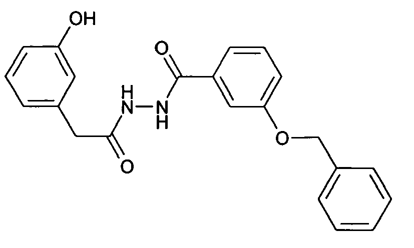 |
| 5 | 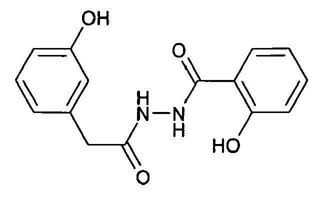 |
| 6 | 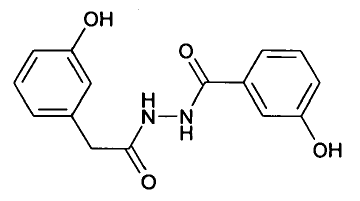 |
| 7 | 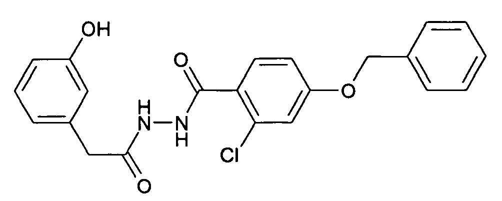 |
| 8 | 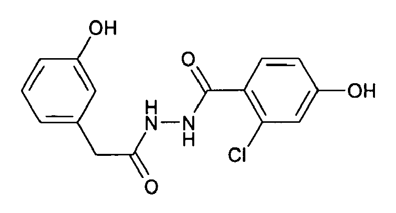 |
| 9 | 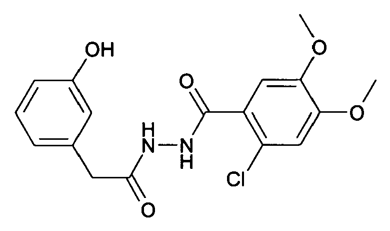 |
| 10 | 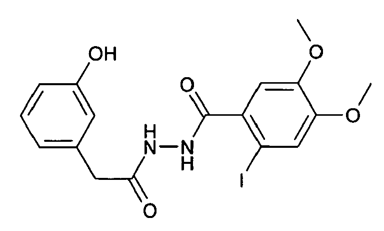 |
| 11 | 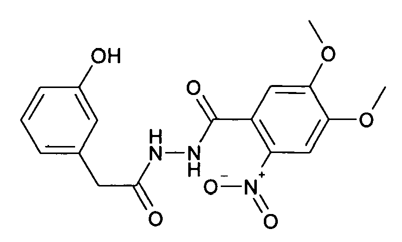 |
| 12 | 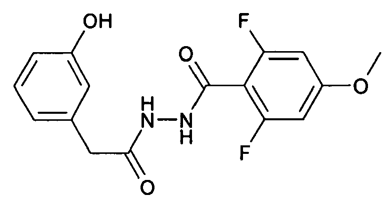 |
| 13 | 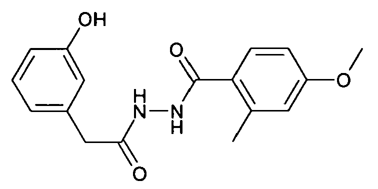 |
| 14 | 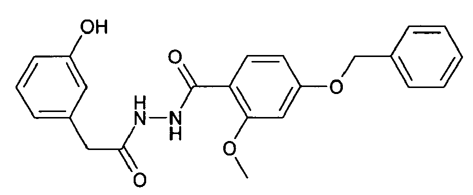 |
| 15 | 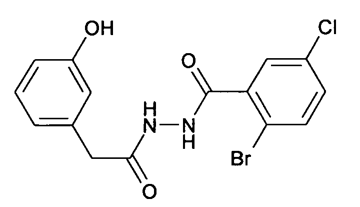 |
| 16 | 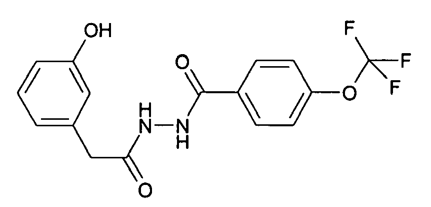 |
| 17 | 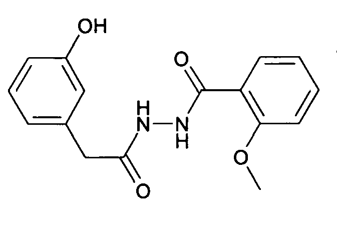 |
| 18 | 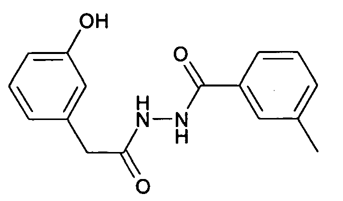 |
| 19 | 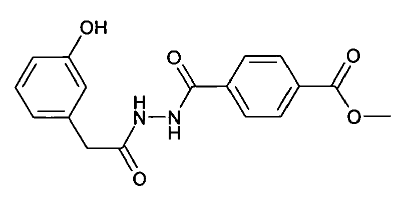 |
| 20 | 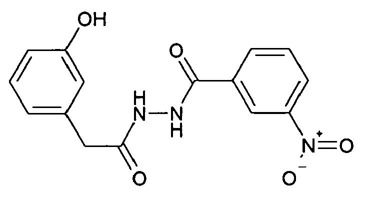 |
| 21 | 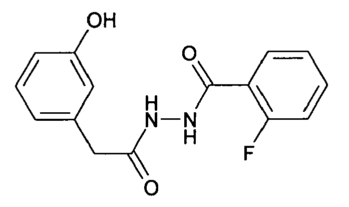 |
| 22 | 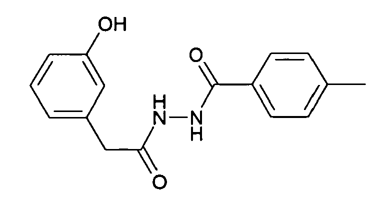 |
| 23 | 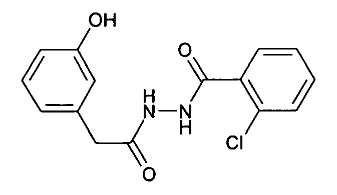 |
| 24 | 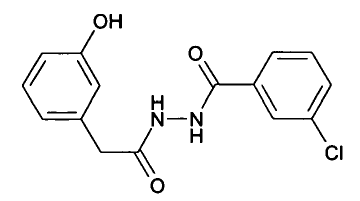 |
| 25 | 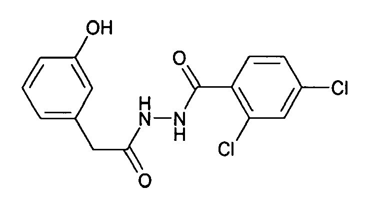 |
| 26 | 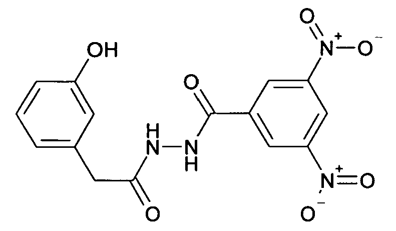 |
| 27 | 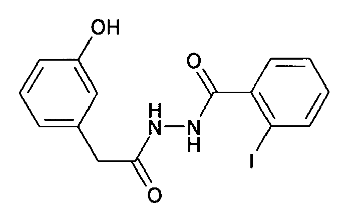 |
| 28 | 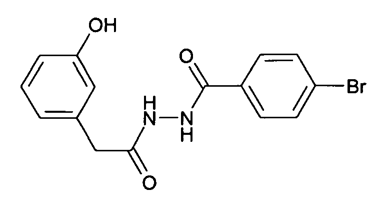 |
| 29 | 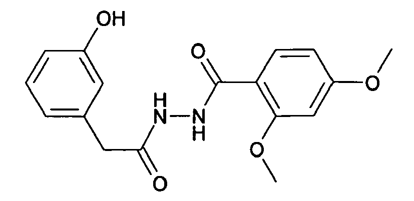 |
| 30 | 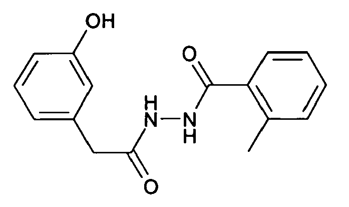 |
| 31 | 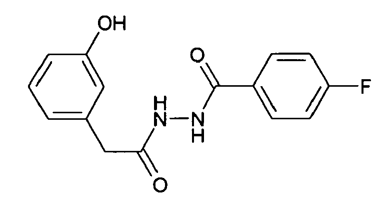 |
| 32 | 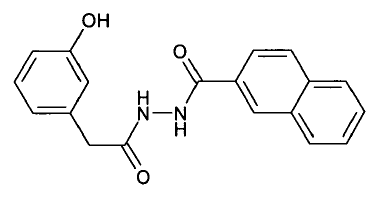 |
| 33 | 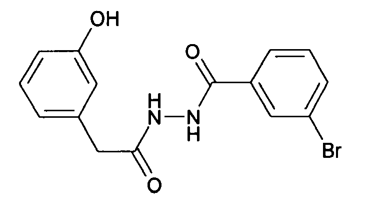 |
| 34 | 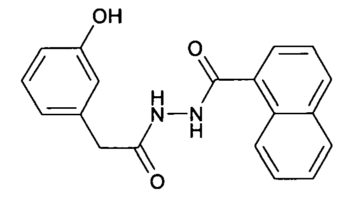 |
| 35 | 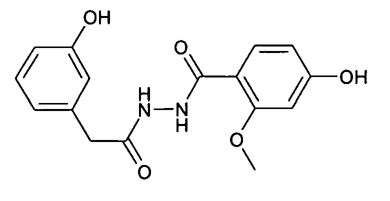 |
| 36 | 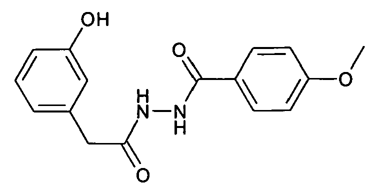 |
| 37 | 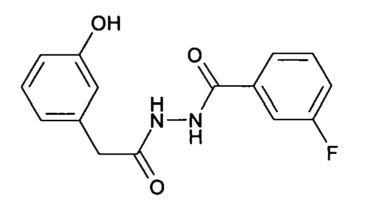 |
| 38 | 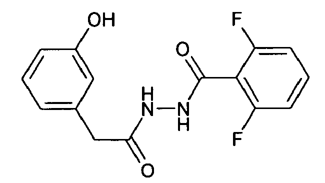 |
| 39 | 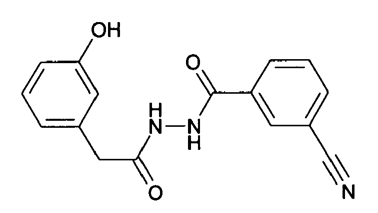 |
| 40 | 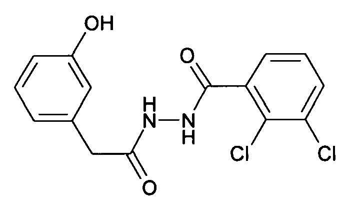 |
| 41 | 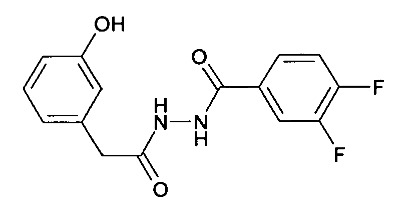 |
| 42 | 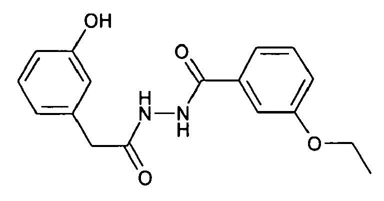 |
| 43 | 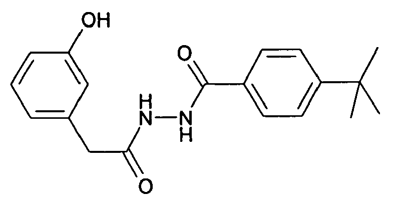 |
| 44 | 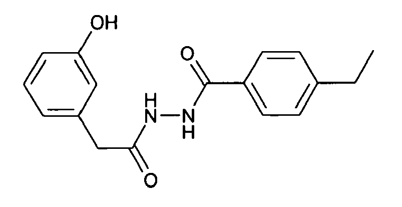 |
| 45 | 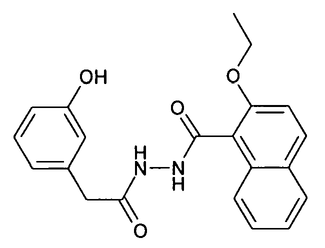 |
| 46 | 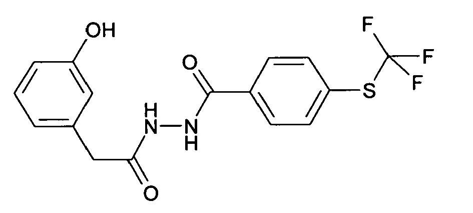 |
| 47 | 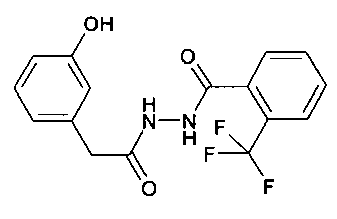 |
| 48 | 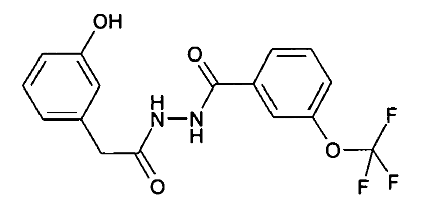 |
| 49 | 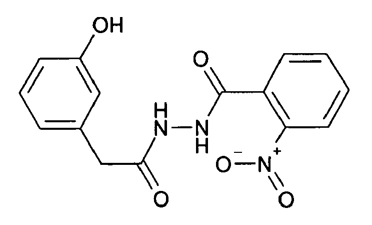 |
| 50 | 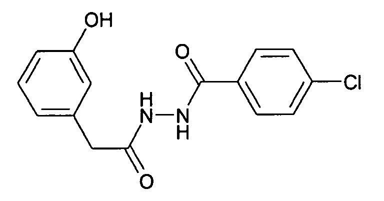 |
| 51 | 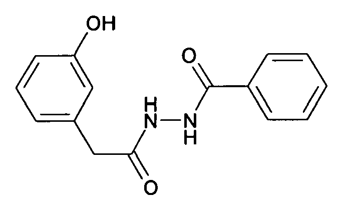 |
| 52 | 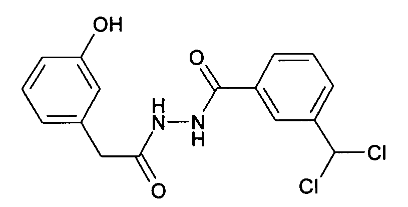 |
| 53 | 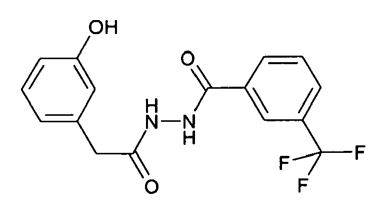 |
| 54 | 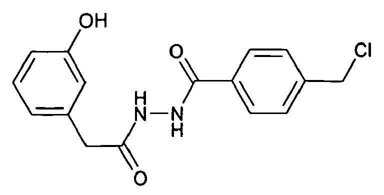 |
| 55 | 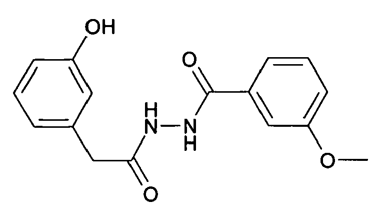 |
| 56 | 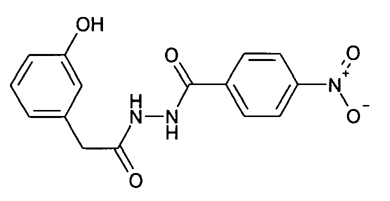 |
| 57 | 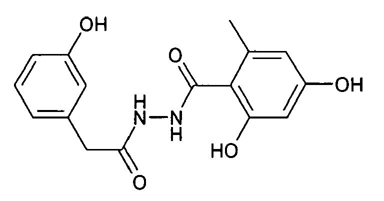 |
| 58 | 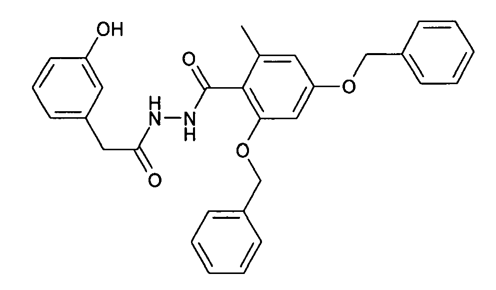 |
| 59 | 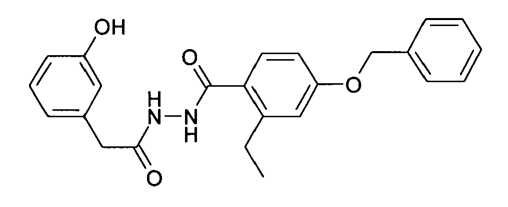 |
| 60 | 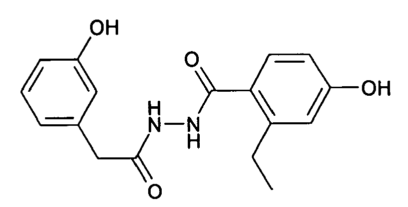 |
| 61 | 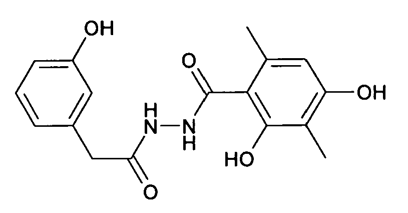 |
| 62 | 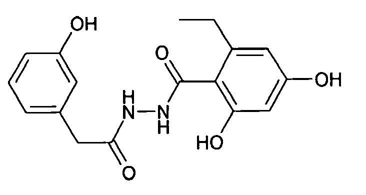 |
| 63 | 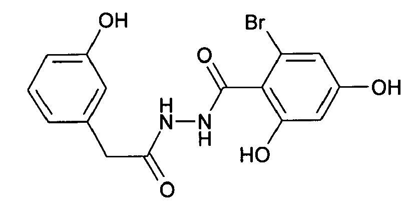 |
| 64 | 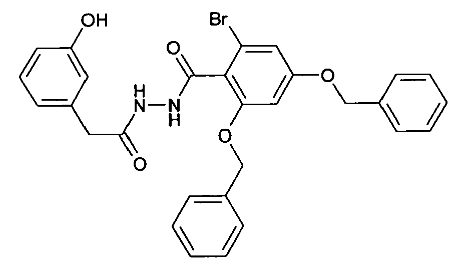 |
| 65 | 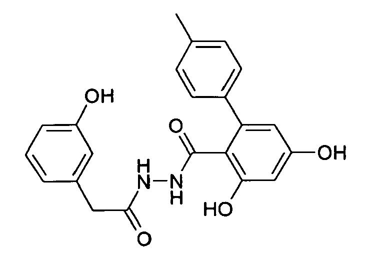 |
| 66 | 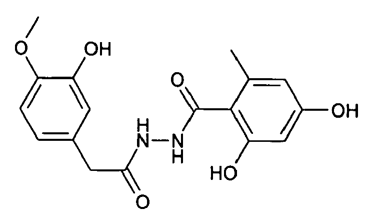 |
| 67 | 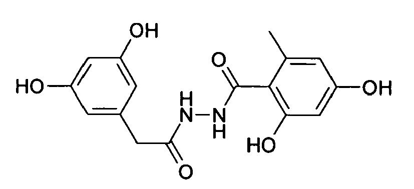 |
| 68 | 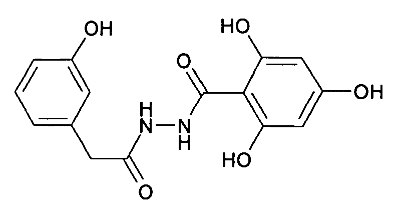 |
| 69 | 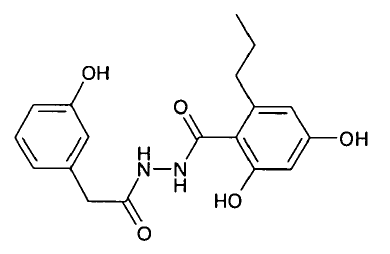 |
| 70 | 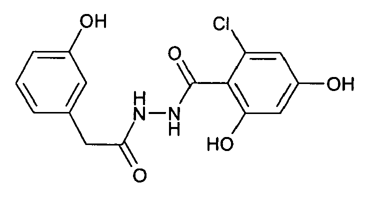 |
| 71 | 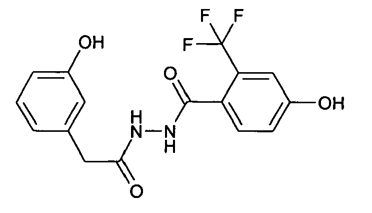 |
| 72 | 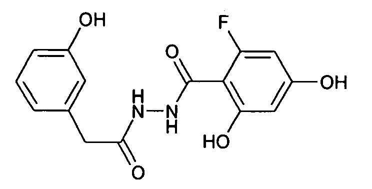 |
| 73 | 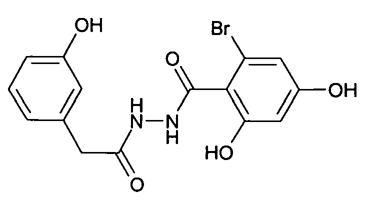 |
| 74 | 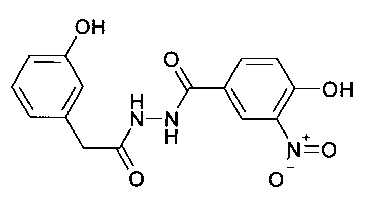 |
| 75 | 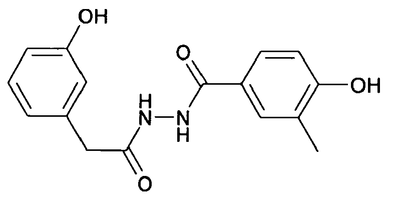 |
| 76 | 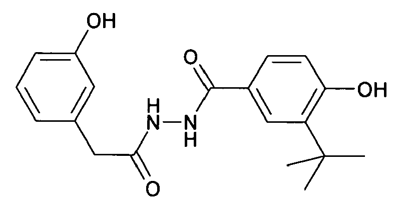 |
| 77 | 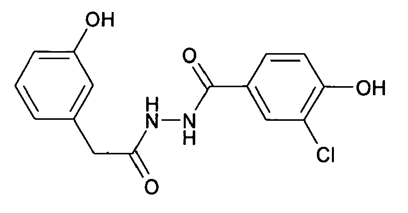 |
| 78 | 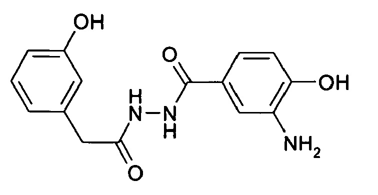 |
| 79 | 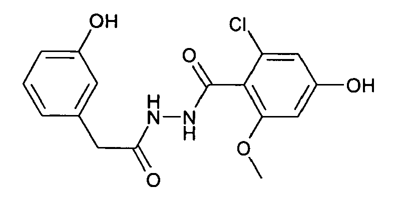 |
| 80 | 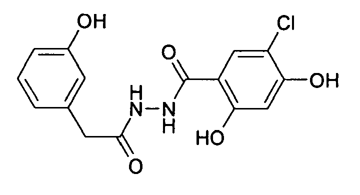 |
| 81 | 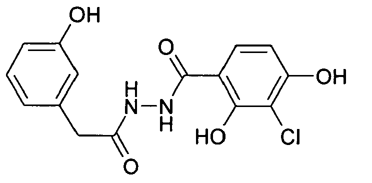 |
| 82 | 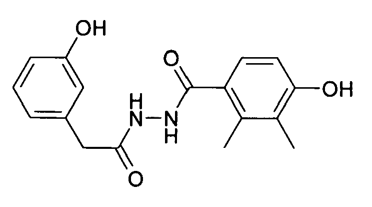 |
| 83 | 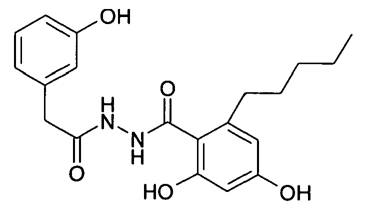 |
| 84 | 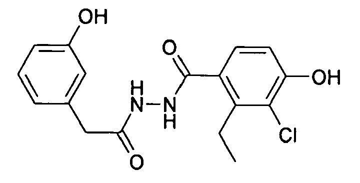 |
| 85 | 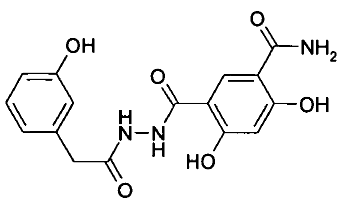 |
| 86 | 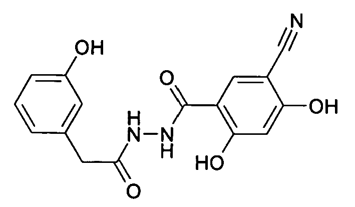 |
| 87 | 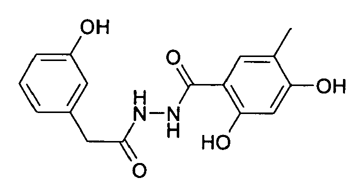 |
| 88 | 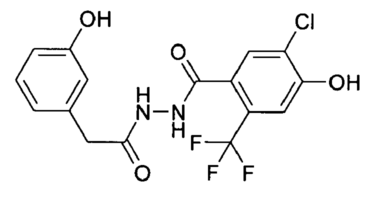 |
ainsi que les dérivés, sels, solvats et stéréoisomères pharmaceutiquement utilisables de ceux-ci, y compris des mélanges de ceux-ci en toutes proportions.

18. Procédé de préparation de composés de la formule I selon les revendications 1-17, ainsi que les dérivés, sels, solvats et stéréoisomères pharmaceutiquement utilisables de ceux-ci, **caractérisé en ce que**
a) un composé de formule II 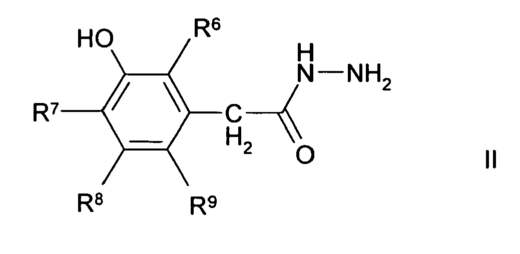 dans laquelle
R⁶, R⁷, R⁸ et R⁹ ont les significations indiquées selon la revendication 1,
est réagi avec un composé de formule III 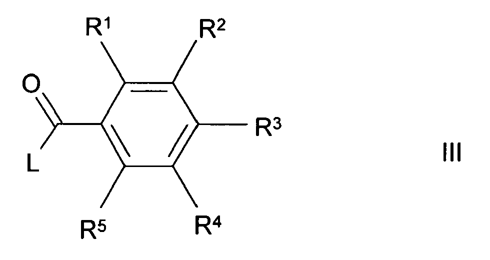 dans laquelle
L désigne Cl, Br, I ou un groupement OH libre ou modifié de manière fonctionnelle et réactive et
R¹, R², R³, R⁴ et R⁵ ont les significations indiquées selon la revendication 1,
ou
b) un composé de formule IV 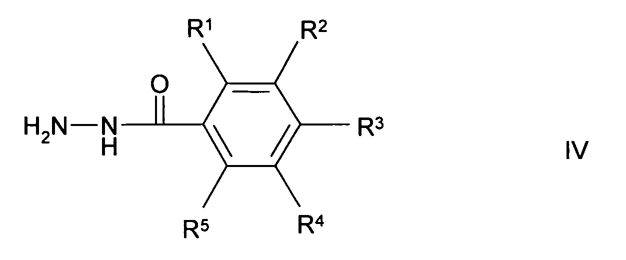 dans laquelle
R¹, R², R³, R⁴ et R⁵ ont les significations indiquées selon la revendication 1,
est réagi avec un composé de formule V 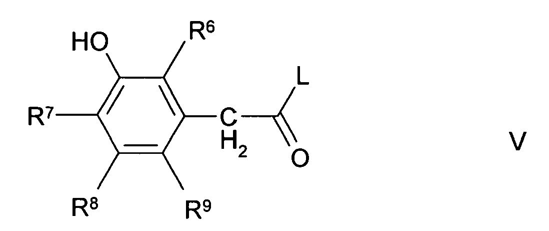 dans laquelle
L désigne Cl, Br, I ou un groupement OH libre ou modifié de manière fonctionnelle et réactive et
R⁶, R⁷, R⁸ et R⁹ ont les significations indiquées selon la revendication 1,
ou
c) un radical R¹, R², R³, R⁴, R⁵, R⁶, R⁷, R⁸ et/ou R⁹ dans un composé de formule I est converti en un autre radical R¹, R², R³, R⁴, R⁵, R⁶, R⁷, R⁸ et/ou R⁹
par clivage d'un éther via une hydrolyse ou une hydrogénolyse,
et/ou une base ou un acide de formule I est converti(e) en l'un de ses sels.

19. Médicaments comprenant au moins un composé selon les revendications 1-17 et/ou des dérivés, solvats et stéréoisomères pharmaceutiquement utilisables de celui-ci, y compris des mélanges de ceux-ci en toutes proportions, et éventuellement des excipients et/ou adjuvants.

20. Utilisation de composés selon les revendications 1-17, et des dérivés, solvats et stéréoisomères pharmaceutiquement utilisables de ceux-ci, y compris des mélanges de ceux-ci en toutes proportions, pour la préparation d'un médicament destiné au traitement et/ou à la prophylaxie de maladies dans lesquelles l'inhibition, la régulation et/ou la modulation de la transduction du signal kinase joue un rôle.

21. Utilisation selon la revendication 20, dans laquelle la kinase est la SGK.

22. Utilisation selon la revendication 21 de composés selon les revendications 1-17, et des dérivés, solvats et stéréoisomères pharmaceutiquement utilisables de ceux-ci, y compris des mélanges de ceux-ci en toutes proportions, pour la préparation d'un médicament destiné au traitement de maladies qui sont influencées par l'inhibition des SGK par les composés selon les revendications 1-17.

23. Utilisation selon la revendication 22 de composés selon les revendications 1-17, et des dérivés, solvats et stéréoisomères pharmaceutiquement utilisables de ceux-ci, y compris des mélanges de ceux-ci en toutes proportions, pour la préparation d'un médicament destiné au traitement ou à la prévention du diabète, de l'obésité, du syndrome métabolique (dyslipidémie), de l'hypertension systémique et pulmonaire, des maladies cardiovasculaires et rénales, généralement dans tous les types de fibroses et de processus inflammatoires, du cancer, des cellules tumorales, des métastases tumorales, des coagulopathies, de l'excitabilité neuronale, du glaucome, des cataractes, des infections bactériennes et de la thérapie anti-infectieuse, pour augmenter les capacités d'apprentissage et l'attention, et au traitement et à la prophylaxie du vieillissement cellulaire et du stress, et pour le traitement de l'acouphène.

24. Utilisation selon la revendication 23, dans laquelle le diabète est le diabète sucré, la néphropathie diabétique, la neuropathie diabétique, l'angiopathie diabétique et la microangiopathie.

25. Utilisation selon la revendication 23, dans laquelle les maladies cardiovasculaires sont les fibroses cardiaques après un infarctus du myocarde, l'hypertrophie cardiaque, l'insuffisance cardiaque et l'artériosclérose.

26. Utilisation selon la revendication 23, dans laquelle les maladies rénales sont la glomérulosclérose, la néphrosclérose, la néphrite, la néphropathie et l'altération de l'excrétion des électrolytes.

27. Utilisation selon la revendication 23, dans laquelle les fibroses et les processus inflammatoires sont la cirrhose hépatique, la fibrose pulmonaire, la pancréatite fibrosante, les rhumatismes et les arthroses, la maladie de Crohn, la bronchite chronique, la fibrose induite par une radiothérapie, la sclérodermie, la mucoviscidose, la cicatrisation et la maladie d'Alzheimer.

28. Médicaments comprenant au moins un composé selon les revendications 1-17 et/ou des dérivés, solvats et stéréoisomères pharmaceutiquement utilisables de celui-ci, y compris des mélanges de ceux-ci en toutes proportions, et au moins un autre composé actif médicamenteux.

29. Ensemble (kit) constitué de packs distincts
(a) d'une quantité efficace d'un composé selon les revendications 1-17 et/ou de dérivés, solvats et stéréoisomères pharmaceutiquement utilisables de celui-ci, y compris des mélanges de ceux-ci en toutes proportions, et
(b) d'une quantité efficace d'un autre composé actif médicamenteux.
